# EUROPEAN PATENT APPLICATION

(11) **EP 2 221 384 A1**
(43) Date of publication of application: **25.08.2010**
(21) Application number: 08856711.0
(22) Date of filing: 03.12.2008
(51) Int. Cl.: C12P 21/08, C07K 16/00, C12N 15/09, A61K 39/395

(54) **METHOD FOR PRODUCTION OF ANTIBODY**

(30) Priority: 03.12.2007 JP 2007312931; 29.08.2008 JP 2008222215
(71) Applicant: KABUSHIKI KAISYA ADVANCE, Chuo-ku Tokyo 103-8354 (JP)
(72) Inventor: INAGAKI, Takashi, Tokyo 103-8354 (JP); YOSHIMI, Tatsunari, Tokyo 103-8354 (JP)
(74) Representative: Ulmann, Catherine Claire
(86) International application number: PCT/JP2008/072361
(87) International publication number: WO 2009/072660

(57) **Abstract**

A method of generating an antibody comprising the steps of: immunizing a tissue containing cells for immunization in vitro in a culture liquid containing an antigen and a stimulating substance, selecting the immunized cells, and obtaining antibodies from the above selected immunized cells. According to this method of generating an antibody, antibodies can be prepared in a short period of time in large quantities, thereby contributing to the increased use of immunological testing.

## Description

### FIELD OF THE INVENTION

The present invention relates to a method of producing an antibody.

### BACKGROUND ART

As is well known, elucidation of immune functions, the mainstay of biological defense functions, is becoming an increasingly important challenge in the diagnosis and treatment of diseases. For example, among the techniques used in the diagnosis of cancer, lifestyle-related diseases of adult people, such as diabetes mellitus, and various other diseases, techniques capable of realizing even simpler diagnosis, such as an immunochromatography method that visually detects the concentration and quantity of a marker substance deeply involved in diseases have been proposed, and the fields of research reagents, diagnostic reagents, various monitoring reagents, immunological diagnosis and treatment are further expanded. Accordingly, it is evident that stable availability of antibodies in the above methods will become even more important. The technology of making an antibody library using the phage display system has also been developed and even isolation of human antibodies has become possible.

Incidentally, the generation of monoclonal antibodies required in immunological measurement takes about three months from the initial injection (immunizing) of an antigen to mice. Subsequently, the B cell group that is producing the antibodies is removed and subjected to cell fusion with myeloma cells. According to this process, a group of antibody-producing cells (hybridomas) capable of infinite growth can be constructed. From these hybridoma groups, cells that are producing the desired antibodies are selected (cloned), and the cells are used in the production of the antibodies in large quantities. In this cloning step, the hybridoma group is diluted until only one cell is present per one well, and the one cell is cultured. This is grown to a cell concentration that enables the investigation of antibody properties, and the properties of the monoclonal antibodies obtained are examined. Wells that tested positive in this examination are diluted again, and examination similar to the above may be conducted. The step is conducted several times to isolate and obtain a hybridoma that can be used. One cycle of this step may take about two weeks, and thus the overall process may sometimes take not less than about three months. Thus, the generation of monoclonal antibodies requires laborious and time-consuming work, and thus, when antibody production is carried out by a specialist, the cost of generating antibodies becomes very high.

Methods of producing antibodies may be specifically explained with regard to known references: Japanese Unexamined Patent Publication (Kokai) No. 10-282097 discloses a specific method of immunization of an antigen that uses a combination of an adjuvant and a cytokine. Japanese Unexamined Patent Publication (Kokai) No. 2004-121237 describes a method of producing antigen-specific antibodies wherein immortalization of peripheral blood lymphocytes for which the response of producing antigen-specific antibodies has been induced by the method of inducing an antibody-production response is caused by the Epstein-Barr virus, and antigen-specific B cells are isolated to prepare B cells that produce the antigen-specific antibodies, and the B cells are further cultured to produce the antigen-specific antibodies. Japanese Unexamined Patent Publication (Kokai) No. 2006-180708 describes a method of producing antibodies comprising the steps of binding a labelled antigen to the target cells, separating the labelled target cells, preparing the antibody gene, and expressing the antibody gene using an expression vector. Japanese National Publication (Kohyo) No. 2006-516408 also describes a method of producing human high-affinity antibodies from the antibodies of interest via a series of steps.

### DISCLOSURE OF THE INVENTION

Thus, it is an object of the present invention to provide a novel method of producing antibodies that enables the laborious and time-consuming work of antibody production to be carried out in a short period of time and easily.

Furthermore, it is an object of the present invention to realize a method of producing antibodies that is useful in the immunological measurement of the S100A10 protein, currently highlighted as a marker for renal cancer, and in treatment based on the novel methods of generating antibodies. As indicated in Tatsuya Takayama et al., Specific Expression of S100A10 Protein in Renal Cell Carcinoma, Proceedings of Renal Cancer Study Group, 28:9-10, 2005 and the like, the S100A10 protein is also useful as a renal cancer marker, and is highly valuable in the simple diagnosis of renal cancer since the marker can be also obtained from urine.

In order to attain the above objectives, the present invention provides a method of producing an antibody, said method comprising the steps of immunizing in vitro a tissue containing cells for immunization in a culture medium containing an antigen and a stimulating substance, selecting the immunized cells, and obtaining the antibodies from the selected immunized cells.

Thus, the present invention has realized the generation of antibodies in a time of about several tenths of the time normally required by obtaining the antigen-sensitized immune cells in a culture liquid supplemented with a specific stimulating substance in the so-called in vitro immunization method.

More specifically, when a foreign substance (antigen) enters into a body, macrophages and dendritic cells incorporate it into the cell. Cells decompose the foreign substance, and present the decomposed products on the surface of the cell membrane. Then naive T cells approach the decomposed products and recognize the antigen via the T cell receptor (TCR) on the surface of the naive T cells. Through this process, T cells are activated to turn into helper T cells (Th2).

On the other hand, B cells also recognize a foreign substance (antigen) that entered into a body via the B cell receptor (BCR) on the cell surface and incorporate it into the cell. Cells also decompose the antigen incorporated and present the decomposed products on the MHC Class II molecules. At this time, a molecule called CD40 is also expressed simultaneously on the surface of the cell membrane.

The thus formed Th2 and the antigen-presenting B cells approach each other, and recognize each other to switch on B cell activation and antibody class switching. At this time, no such phenomenon appears unless the molecules are activated with the same antigen.

Incidentally, the immune reaction is conducted in vivo in the above case, whereas a method of conducting the immune reaction ex vivo is the so-called in vitro immunization method.

The present invention intends to induce the differentiation of B cells into antibody-producing cells (plasma cells) or memory B cells by adding a stimulating substance in order to obtain the desired antibody-producing cells in the in vitro method. As used herein the term "stimulating substance" is not specifically limited, as long as it can interact with the receptor molecule expressed on the surface of the membrane of immunocompetent cells such as B cells, dendritic cells, and T cells. In the practice of the present invention, as a stimulating substance for cytokine receptors specifically, there can be illustrated IL-1, IL-2, IL-3, IL-4, IL-5, IL-6, IL-7, IL-10, and IL-21, and as a stimulating substance for surface antigens there can be illustrated TGF-β (transforming growth factor-beta), BAFF (B cell activating factor), APRIL (a proliferation-induced ligand), CD40 ligand, CD38 ligand, BCDF (B cell differentiation factor), and BCAF (B cell activation factor), and as a stimulating substance for receptors involved in signal transduction there can be illustrated LPS (lipopolysaccharide) and the like. The concentration used of a stimulating substance may vary depending on the concentration of the cells to be immunized, but for LPS the concentration of 10 ng/mL - 500 µg/mL is preferred, and specifically the concentration of 20-80 µg/mL is recommended. Also, for substances other than LPS, the concentration of 1 ng/mL - 50 µg/mL is generally preferred, and specifically the concentration of 10-40 ng/mL is recommended. An agonist antibody that can substitute the function of these substances can also be used. In the process of differentiation induction, stimulation with IL-4, IL-5, anti-CD38 antibody, or anti-CD40 antibody can induce the maturation of antibody affinity and class switching so as to turn it into antibodies having higher affinity.

Furthermore, using a cell separation instrument such as FACScan based on flow cytometry, etc., the sensitized immune cells may be detected and separated using a technology of staining and selecting the cells of interest.

A staining agent for use in the selection and detection of immune cells of the present invention is not specifically limited. For example, trypan blue is used as a staining agent in the detection of B cells that propagate and survive, and furthermore an antigen or antibody labelled with a fluorophore such as FITC and PE for use in flow cytometry, a fluorescent probe, such as Fura-2, that detects the calcium concentration in the cell, and a DNA staining agent represented by ethidium bromide that stains DNA in the cell and the like may be used as appropriate, as long as they can stain the immune cells of interest.

In selecting cells in which class switching or affinity maturation has occurred, a staining agent similar to those described above can be used as appropriate.

As methods of detecting and/or selecting the stained cells, in addition to cell separation equipment such as flow cytometry, a blood cell counting device such as a hemacytometer, an electrophoresis device, a centrifugation device, a magnetic bead device, a micro flow path device and the like is preferably used.

Also, though the selected cells and myeloma cells may be fused to obtain a hybridoma, a hybridoma may not necessarily be obtained.

Furthermore, the present invention can be carried out in such a manner that, in order to separate cells to obtain an antibody gene from tissue, immune cells are selected by an in vitro immunization step, and from the immunized cells an antibody gene may be formed by the PCR technology, which is introduced into the host cell and cultured for expression to produce the antibodies in large quantities, and according to the present technology, work efficiency can be enhanced since the post-immunization cells are obtained from tissue fragments.

A large quantity of antibodies can be prepared in a short period of time by amplifying an antibody gene obtained from the cells by the PCR (polymerase chain reaction) method and combining a step of obtaining an antibody from host cells, such as Escherichia coli preferably by colonizing it. As host cells, in addition to Escherichia coli, there can be illustrated streptomyces, Bacillus subtilis, yeast, CHO cells, COS cells, HEK-293 cells and the like.

The expression vector is not specifically limited as long as it can be expressed in the host cell. As the expression vector, there can be illustrated bacterial plasmid-derived, yeast plasmid-derived, chromosome, episome, virus-derived, retrovirus-derived vectors, and the like.

In addition to the problem described above, the present inventors have also addressed the problem of possible separation of an antibody gene without requiring a hybridoma-forming step as a preparatory process until screening, even if immunized cells were selected and obtained in vitro.

The present inventors have obtained a finding that in an in vitro immunization method, the induction of differentiation into memory B cells or plasma cells, affinity maturation and class switching of an antibody thought to be formed in the process can be realized by the addition of a given stimulating substance, and thereby have completed the present invention.

Conventionally, in the generation of a high-affinity antibody, an antigen was repeatedly immunized in vivo to induce differentiation into memory B cells or plasma cells, and the affinity maturation and/or class switching of an antibody thought to be generated in the process was utilized. A high-affinity antibody can be obtained by forming a hybridoma from the differentiated immunized cells followed by screening. On the other hand, in the case of ex vivo, the generation was principally carried out using the method comprised of constructing a library in which various mutations have been introduced into an antibody gene obtained from a hybridoma or a commercial library, etc., by random mutation introduction using PCR, and, after its integration into a phagemid vector, screening a strong antibody by the panning method.

However, the present inventors have found that the induction of differentiation into memory B cells or plasma cells and the affinity maturation and/or class switching of an antibody thought to be generated in the process can also be effected by the supply of a specific stimulating substance ex vivo under a given environment. Furthermore, since the introduction of a stimulating substance enables the differentiation of B cells into memory B cells or plasma cells, the method in the in vitro immunization has enabled the immunization with B cells alone which could only had been realized in the conventional spleen cells or in the co-culture of B cells and T cells, and also enabled to minimize the amount of antigen, as in the conventional immunization method, to about 1/1000 - 1/10000 that in the in vivo immunization.

In addition, a stimulating substance for use in the present invention also realized the induction of the expression of a protein involved in the affinity maturation and class switching of an antibody, resulting in a rapid and assured class switching from IgM to IgG of an antibody.

In brief, the present invention realizes the implementation of the so-called in vitro immunization in a time of about several tenths of the time required when an animal was normally immunized by obtaining immunized cells by sensitization with an antigen in a culture liquid in which a specific stimulating substance has been added to the cells obtained by grinding tissue fragments.

In the practice of the above method of generating antibody according to the present invention, a method of generating an immune reaction in vivo or in vitro can be advantageously carried out by the method described above.

With regard to the in vitro immunization method of the present invention, the relationships between various stimulating substances and a variety of differentiation and induction, etc., are explained below.

For an antigen as used herein, onetime immunization after preparation of a tissue fragment for immunization is sufficient but it may be carried out for multiple times with intervals of 2-3 days. The antigen concentration at this time may preferably be 1 pmol to 1 mmol, and for example 1 nmol may be mentioned.

For the purpose of assisting antigen stimulation, a substance that stimulates TLR (Toll like receptor) represented by LPS may be added. The addition of this stimulating substance may preferably be carried out at each time of antigen stimulation, and the amount added may preferably be at a concentration of 10 ng/mL to 500 µg/mL, most preferably 20-80 µg/mL.

As a stimulating substance as used herein for inducing differentiation into memory B cells or plasma cells and inducing the affinity maturation and/or class switching of an antibody thought to be generated in the process, an stimulating substance mentioned above may be used without limitation, and specifically IL-4, IL-5, anti-CD38 antibody, and anti-CD40 antibody are preferred. The concentration added is preferably 1 ng/mL to 50 µg/mL for IL-4 and Il-5, most preferably 10 ng/mL to 40 ng/mL, and 1 ng/mL to 50 µg/mL for anti-CD38 antibody and anti-CD40 antibody, most preferably 100 ng/mL to 10 µg/mL. The stimulating substance may be added every time antigen stimulation is carried out.

As cells that constitute a tissue fragment for immunization conducted in vitro of the present invention, there can be illustrated those described above, for example cells derived from organs or tissues of immune animals such as a mouse, a rat, a guinea pig, and a rabbit, immunocompetent cells prepared by differentiating iPS cells or ES cells, immunocompetent cells prepared by differentiating cultured cell lines, and the like.

As antigens for use in the present invention, there can be illustrated proteins derived from animals other than mice such as h(human)S100A0, he(hen)EL, h(human)Ras, h(human)rap74, h(human)TOP02B, b(bovine)SA and b(bovine)Casein, mouse-derived proteins, such as m(mouse)S100A10, mSA, and mmapk1, and peptides (four types: 7 mer, 10 mer, 16 mer, 20 mer), and the like. Furthermore, in accordance with the practice of the present invention, low molecular weight compounds (a fluorescent substance such as rhodamine, FITC) can also be used which generally cannot be used in the conventional method of obtaining antibodies.

In accordance with the present invention, due to the introduction of a stimulating substance depending on the intended in vitro use, T cells may not be required; due to the step of inducing the differentiation of B cells to antibody producing cells, the use of a low molecular weight antigen can be realized; and furthermore immunized cells that enable to obtain a preferred antibody by class switching and antibodies by affinity maturation can be obtained.

As described above, furthermore, a cell separation instrument based on flow cytometry such as FACScan, and the like may be used so as to detect and separate sensitized immunized cells using a method of staining and selecting the cells of interest.

Furthermore, since the size of immune cells obtained may be greater than other cells, and when affinity was maturated, cells may be larger, and thus separation based on cell size may be effected. Also, as a selecting method based on the size of the cells, a technique described in Japanese Unexamined Patent Publication (Kokai) No. 2007-175684, etc., is preferably be used.

Staining agents to be used in the separation and detection of immunized cells of the present invention are as described above. Also, in the separation of cells that underwent class switching or affinity maturation, as described above, similar staining agents can be used.

Furthermore, the present invention may take a constitution in which, as described above, cells for preparing an antibody gene may be obtained by selecting immunized cells from a tissue by an in vitro immunization step, from which immunized cells, an antibody gene may be formed using the PCR method, and the gene may be introduced into host cells, cultured and expressed for the production of a large quantity of antibody, and according to said technology, work efficiency of obtaining the cells after immunization from a tissue fragment can be enhanced. Even when the gene of cells from the tissue fragment was separated without selecting the immunized cells, it is possible to obtain only the gene of interest and to adopt a method of extracting the antibody gene in a similar manner to when the immunized cells were selected, since the primers used in PCR are specific for the antibody gene.

As described above, it is possible to generate a large quantity of antibodies in a short period of time by amplifying the antibody gene obtained from the cells by the PCR method, and furthermore by combining preferably a step of colonizing the cells with the host cells such as Escherichia coli to obtain the antibody. The host cells and expression vectors that can be used are as described above.

As a method of selecting an antibody according to the present invention, there can be illustrated a method of displaying an antibody on the surface of phage, Escherichia coli, yeast, animal cells etc., the 2-hybrid method using yeast, Escherichia coli etc., a method of displaying DNA or mRNA, such as the IVV method and the ribosome display method, the BIOCORE method utilizing surface plasmon resonance, the ELISA method, the Western blot method, a method (MACS) of immobilizing an antibody or an antigen on magnetic beads and selecting it using magnetic force, and the like. The method of separation is not specifically limited, as long as it is a technology for examining the protein-protein interaction.

In accordance with the present invention, as can be understood from the following detailed explanation, immunized cells of interest can be selected and harvested in a short period of time, and furthermore through the generation of antibodies by a recombinant technique, the antibodies can be quickly produced and harvested. These immunized cells are specifically preferred for screening.

### BRIEF EXPLANATION OF THE DRAWINGS

Figure 1A is a photograph showing the electrophoresis of the total RNA prepared, and Figure 1B is an explanatory figure thereof,
Figure 2A is a photograph showing the electrophoresis of the cDNA prepared, and Figure 2B is an explanatory figure thereof,
Figure 3A is a photograph showing the electrophoresis of the scFv PCR product prepared, and Figure 3B is an explanatory figure thereof,
Figure 4A is a photograph showing the electrophoresis of the Escherichia coli clone prepared, and Figure 4B is an explanatory figure thereof,
Figure 5A is a drawing of the analysis by the SDS-PAGE method of the purified MBP-scFv protein, and Figure 5B is an explanatory figure thereof,
Figure 6 is a graph that plots the relation between the antigen and IgG1 positivity,
Figure 7 is a graph that plots the relation between the marker gene and the amount of mRNA expressed,
Figure 8 is a graph that plots the result of cytotoxicity testing of cytokines as a relation between the stimulating substance and the specific growth rate,
Figure 9A and Figure 9B are each graphs that plot the relation between the stimulating substance and the specific growth rate,
Figure 10A, Figure 10B, Figure 10C and Figure 10D are each graphs that plot the result of the amount expressed of Blimp-1, the amount expressed of Xbp-1, the amount expressed of Bcl-6 and the amount expressed of AID in the single stimulation,
Figure 11A and Figure 11B are each graphs that plot the result of the amount expressed of Blimp-1 and the amount expressed of Xbp-1 in the combined stimulation and uring plasma cell differentiation,
Figure 12A and Figure 12B are each graphs that plot the result of the amount expressed of Bcl-6 and the amount expressed of AID in the combined stimulation and during somatic mutation,
Figure 13 is a graph that plots the result of the amount expressed of Blimp-1 in the combined stimulation and during plasma cell differentiation,
Figure 14 is a graph that plots the result of the amount expressed of Xbp-1 in the combined stimulation and during plasma cell differentiation,
Figure 15 is a graph that plots the result of the amount expressed of Bcl-6 in the combined stimulation and during somatic mutation,
Figure 16 is a graph that plots the result of the amount expressed of AID in the combined stimulation and during somatic mutation,
Figure 17A and Figure 17B are each graphs that plot the amount expressed of a marker at the upper limit and the lower limit of cytokine when 1 nmol of antigen was immunized and when 0.1 nmol of antigen was immunized,
Figure 18A and Figure 18B are each graphs that plot the result of investigating B cell differentiation in terms of the ratio of B cells in the spleen cells and the ratio of plasma cells in the spleen cells,
Figure 19A, Figure 19B, Figure 19C and Figure 19D are each graphs that plot the result of investigating B cell differentiation in terms of the ratio of T1-B cells in the B cells, the ratio of T2-B cells in the B cells, the ratio of B2 cells in the B cells and the ratio of activated-B cells in the B cells,
Figure 20A, Figure 20B, Figure 20C and Figure 20D are each graphs that plot the amount expressed of Blimp-1, the amount expressed of Xbp-1, the amount expressed of Bcl-6 and the amount expressed of AID in the investigation of B cell differentiation,
Figure 21 shows the ELISA result of anti-Hen Egg Lysozyme/MBP-scFv obtained by the immunization procedure on splenocytes, and is a graph comparing β-casein,
   S100A2, S100A14 and Hen Egg Lysozyme using an ELISA plate coated with each of them to examine antigen specificity,
Figure 22 shows the ELISA result of anti-Hen Egg Lysozyme/MBP-scFv obtained by the immunization procedure on B cells, and is a graph comparing β-casein, S100A2, S100A14 and Hen Egg Lysozyme using an ELISA plate coated with each of them to examine antigen specificity,
Figure 23 shows the ELISA result of anti-Hen Egg Lysozyme/MBP-scFv/EGFP in which EGFP has been fused to scFv via G1-linker and G5-linker, and is a graph comparing β-casein, S100A2, S100A14 and Hen Egg Lysozyme using an ELISA plate coated with each of them to examine antigen specificity,
Figure 24 shows the ELISA result of anti-β-casomorphin 7/MBP-scFv, anti-angiotensin I/MBP-scFv and anti-PTH/MBP-scFv, and is a graph comparing β-Cas7β-casomorphin 7 (b-Cas7), angiotensin I (Ang I) and PTH using an ELISA plate coated with each of them to examine antigen specificity,
Figure 25 is a graph that plots the result of evaluating an anti-mouse protein antibody obtained in terms of absorbance (mAlbumin ELISA),
Figure 26 is a graph that plots the result of evaluating an anti-mouse protein antibody obtained in terms of the rate of appearance of positive clones (mAlbumin positivity),
Figure 27 is a graph that plots the result of evaluating an anti-mouse protein antibody obtained in terms of absorbance (mS100A10 ELISA 1 nmol antigen),
Figure 28 is a graph that plots the result of evaluating an anti-mouse protein antibody obtained in terms of absorbance (mS100A10 ELISA 1 nmol antigen),
Figure 29 is a graph that plots the result of evaluating an anti-mouse protein antibody obtained in terms of the rate of appearance of positive clones (mS100A10 positivity), and
Figure 30 is a photograph of electrophoresis showing an anti-low molecular weight compound antibody obtained.

### BEST MODE FOR CARRYING OUT THE INVENTION

Subsequently, the preferred embodiments of the present invention will be explained. It should be understood that the present invention is not limited to the specific embodiments described below.

The present invention is intended to obtain immunized cells by extracting an organ from an animal for immunization, such as a mouse and a rat, selecting a group of cells containing the immunized cells from the organ, and sensitizing them with an antigen in a culture liquid containing a stimulating substance to obtain the immunized cells.

The immunized cells are subjected to cell separation equipment such as FACScan or other separation means such as cell staining to separate B cells producing the antibody of interest from the lymphocytes. The selected B cells can be immortalized via fusion with myeloma cells to produce hybridomas, and, after cloning, antibodies and an antibody gene can be obtained, but the step of generating hybridoma is not always necessary.

The antigen need not be specified if, from the selected cells or the hybridoma, gene is extracted, and subjected to a PCR method to obtain the genes of H chains and L chains, which are modified to a single chain antibody scFv to turn into a plasmid so as to be expressed in Escherichia coli, and the antibody can be obtained by expressing it in Escherichia coli. Among the antigens, S100A10, for example, can be used as a marker for renal cancer that can use urine as the sample, and thus the present invention is preferred as a method of generating an antibody for the immunological diagnosis of renal cancer.

### [On the generation of human antibody derived from a mouse]

In accordance with the present invention, the step of generating a mouse-derived human antibody may be further combined. The exemplary step of obtaining human antibodies is shown below.

### 1. A method of using spleen cells derived from an immunized humanized mouse

An immunized humanized mouse has been reported in which all the antibody genes in the mouse spleen cells were replaced with human-derived genes. Since the cells are derived from a mouse, except the antibody gene, they can be used as they are in the in vitro immunization method. All the antibodies obtained in this method are human antibodies.

### 2. A method of humanizing an antibody

From the gene obtained, the amino acid sequence of the antibody may be determined. This amino acid sequence is compared with the amino acid sequence library of human antibodies. Specifically the framework portion of antibody other than the hypervariable region (complementarity determining region) may be compared so as to select a sequence with a high homology from the amino acid sequence library of human antibody. The hypervariable region of the antibody gene obtained is integrated into the selected frame sequence for humanization. At this stage, the antibodies are humanized mouse antibodies.

For the full-length antibody, the amino acid sequence of the antibody may be determined from the gene obtained. The amino acid sequence may be compared with the amino acid sequence library. By comparing the full-length of the antibodies containing the hypervariable region, a sequence with a high homology may be selected from the amino acid sequence library of human antibodies. By expressing the selected sequence in the cell, full-length human antibodies can be obtained. The hypervariable region of the selected sequence may be altered by gene recombinant technology to a sequence completely identical with the original sequence to prepare full-length human antibodies.

The present invention is summarized as follows.
(1) A method of generating an antibody comprising the steps of:
   immunizing a tissue containing cells for immunization in vitro in a culture liquid containing an antigen and a stimulating substance,
   selecting the immunized cells, and
   obtaining an antibody from the above selected immunized cells.
(2) The method of generating an antibody according to the above item 1, the method further comprising the steps of obtaining an amplified antibody gene by a PCR amplification means and then expressing and preparing the antibody or a protein similar to the antibody by host cells.
(3) The method of generating an antibody according to the above item 1 wherein the tissue is an animal for immunization.
(4) The method of generating an antibody according to the above item 1 wherein the selected cells are cells that underwent class switching or affinity maturation.
(5) The method of generating an antibody according to the above item 1 wherein the selection of the immunized cells is based on flow cytometry or staining.
(6) The method of generating an antibody according to the above item 1 wherein the stimulating substance is a substance that stimulates a cytokine receptor, a surface antigen or a receptor involved in signal transduction expressed on immunocompetent cells.
(7) The method of generating an antibody according to the above item 1 wherein the antigen is a peptide.
(8) The method of generating an antibody according to the above item 1 wherein the antigen is a protein belonging to the S100 family, and a single chain antibody scFv (single chain variable fragment) or an antibody IgG1 (immunoglobulin G1) are obtained.
(9) The method of generating an antibody according to the above item 1 wherein said antigen is a protein belonging to the S100A10 family, and a single chain antibody scFv (single chain variable fragment) or an antibody IgG1 (immunoglobulin G1) are obtained.
(10) The method of generating an antibody according to the above item 6 wherein the stimulating substance is at least one of IL-4 or Il-5 as a stimulating substance for a cytokine receptor, anti-CD38 antibody or anti-CD40 antibody as a stimulating substance for a surface antigen, and a LPS (lipopolysaccharide) as a stimulating substance for a receptor involved in signal transduction.
(11) The method of generating an antibody according to the above item 1 wherein an antibody is generated by immunizing in vitro a tissue containing cells for immunization in a culture liquid containing one or more than one stimulating substance selected from IL-4, IL-5, anti-CD38 antibody, anti-CD40 antibody and LPS (lipopolysaccharide).
(12) The method of generating an antibody according to the above item 1 further comprising the steps of:
   determining the amino acid sequence of said selected antibody, and
   comparing this amino acid sequence with an amino acid sequence library of human antibody to select or modify a sequence with a high homology from the amino acid sequence library of human antibody to generate a humanized antibody.

### EXAMPLES

The present invention will now be explained more specifically with reference to the following examples. It should be understood that these examples do not limit the present invention in any way.

### Example 1

The example describes a method of generating antibody IgG1 using the S100A10 protein as the antigen.

### Extraction of spleen cells (splenocytes)

After visual identification, mouse spleens were extracted and washed. Spleens from two animals were placed on a mesh, mashed with a cell scraper, centrifuged (1300 rpm × 3 min, 4°C), and the cells were collected. Then, after suspending the cells in 3 mL of ACK lysing buffer, 10 mL of PBS(-) was added thereto. The cells were centrifuged (1300 rpm × 3 min, 4°C) and collected, which were then suspended in 6 mL of RPMI1640(-). Through a cell strainer, insoluble fats etc. were removed.

### in vitro immunization

The concentration of the S100A10 protein antigen was determined using an assay kit (Dc Protein Assay kit, manufactured by BioRad). As the standard, BSA (manufactured by Sigma) was used. A predetermined amount (5 µg or 20 µg) of the S100A10 protein antigen of a known concentration was aliquoted, to which 100 µl (50 µl per mouse) of 1 mg/mL acetylmuramyl-L-alanyl-D-isoglutamine was added, and allowed to stand at room temperature for 15 minutes. 6 mL each of RPMI1640 medium (40% FBS) was added. A stimulating substance (IL-4 at a final concentration of 10 ng/mL, IL-5 at a final concentration of 10 ng/mL, anti-CD38 antibody at a final concentration of 1 µg/mL, anti-CD40 antibody at a final concentration of 1 µg/mL, LPS at a final concentration of 40 µg/mL) was added. The total amount was plated on a 10 cm dish, and incubated in a 37°C CO₂ incubator for 4 days. After culturing, the cells were collected by centrifugation (2000 rpm, 5 minutes, 4°C), and stained with Trypan Blue Stain 0.4% (manufactured by GIBCO) to count the number of spleen cells after immunization on a hematocytometer (manufactured by AS ONE Corporation). The number of cells was 1.33 × 10⁷ when immunized with 5 µg of S100A10 antigen and 0.71 × 10⁷ when immunized with 20 µg of S100A10 antigen. The cells were washed (10 mL × 3 times) with Medium B (Clona Cell HY kit, manufactured by StemCell Technology) to prepare to 1.00 × 10⁸ cells/mL.

### Preparation of melanoma cells

PAI cells (mouse-derived myeloma cells) had been subcultured using Medium A (Clona Cell HY kit, manufactured by StemCell Technology). The cells were washed (10 mL × 3 times) using Medium B and counted using the same instrument as above. The number of the cells is preferably 5 × 10⁷ cells at the time of cell fusion.

### Cell fusion

Splenocytes and melanoma cells were mixed at 2:1 and centrifuged for collection. After tapping, the cells were mixed with a PEG (polyethylene glycol) solution: 66.5 µl of the PEG (polyethylene glycol) solution when immunized with 5 µg of the S100A10 antigen, and 35.5 µl (500 µl relative to 1 × 10⁸ splenocytes) of the PEG solution when immunized with 20 µg of the S100A10 antigen.

After centrifuging and collecting the cells, Medium B was added dropwise to the cells: 665 µl of Medium B when immunized with 5 µg of the S100A10 antigen and 355 µl (5 ml relative to 1 × 10⁸ splenocytes) of Medium B when immunized with 20 µg of the S100A10 antigen.

Medium C (Clona Cell HY kit, manufactured by StemCell Technology) was added dropwise to them: 665 µl of Medium C when immunized with 5 µg of the S100A10 antigen and 355 µl (5 ml relative to 1 × 10⁸ splenocytes) of Medium C when immunized with 20 µg of the S100A10 antigen. This solution was gently added to Medium C: 5.32 mL of the solution was added to Medium C when immunized with 5 µg of the S100A10 antigen and 2.84 ml (40 ml relative to 1 × 10⁸ splenocytes) of the solution was added to Medium C when immunized with 20 µg of the S100A10 antigen, and cultured (for one day) (in a 15 mL centrifuge tube) in a 37°C CO₂ incubator.

On the day of cell fusion, Medium D (Clona Cell HY kit, manufactured by StemCell Technology) was transferred to a 4°C incubator for dissolution. After dissolution, it was mixed well and returned to room temperature.

### Cloning

The cells were centrifuged and collected, and added to Medium C: 1.33 mL of Medium C when immunized with 5 µg of the S100A10 antigen, and 0.71 ml (10 ml relative to 1 × 10⁸ splenocytes) of Medium C when immunized with 20 µg of the S100A10 antigen, and this was suspended in Medium D: 11.97 mL of Medium D when immunized with 5 µg of the S100A10 antigen and 6.39 ml (90 ml relative to 1 × 10⁸ splenocytes) of Medium D when immunized with 20 µg of the S100A10 antigen. After incubating at 37°C for 15 minutes, the cells were plated on a 10 cm dish. Furthermore, they were incubated in a 37°C CO₂ incubator (for 10-14 days).

### Harvest

The cell colonies were visually counted, and the total amount was transferred to a 96-well plate, to which 200 µl of Medium E (Clona Cell HY kit, manufactured by StemCell Technology) was added. Then, it was cultured (4 days) in a 37°C CO₂ incubator. The supernatant (150 µl) was collected, and identified by S100A10 ELISA.

### Preparation of a S100A10 ELISA plate

The S100A10 antigen was diluted in PBS(-) to 5 µg/mL, and 100 µl each of it was added to a 96-well plate (Nunc-Immuno module F8 maxisorp, manufactured by Nunc). After allowing this to stand at 4°C for one day, the supernatant was discarded, and 200 µl each of 0.1% BSA/PBS(-) was added. After allowing this to stand at room temperature for 1 hour, it was washed three times with PBS(-) to prepare a S100A10 ELISA plate.

### S100A10 ELISA

To the S100A10 ELISA plate, 150 µl of the culture supernatant obtained in the above Harvest section was added, and allowed to stand for 1 hour in a 37°C incubator. The supernatant was discarded, washed three times with PBS(-), and 50 µl of a secondary antibody (anti-mouse IgG1-HRP, manufactured by Funakoshi Corporation) was added, and allowed to stand in a 37°C incubator for 1 hour. The supernatant was discarded, washed three times with PBS(-), 100 µl of a TMB solution was added, and allowed to stand in a 37°C incubator for 10 minutes. Immediately thereafter, 100 µl of 1N HCl (manufactured by Wako Pure Chemical Industries, Ltd.) was added to stop the reaction. Then, the absorbance of each well was measured at 450 nm using a microplate reader (Model 550, manufactured by BioRad). The results obtained are shown in the following Table 1.

**Table 1**

| Antigen | Amount Immunized (µg) | No. of splenocytes after immunization (x 10⁷ cells) | No. of hybridomas | | | Growth after cloning | | No. of IgG1- expressing lines |
|---|---|---|---|---|---|---|---|---|
| | | | Large colonies | Small colonies | Total | ++ | + | |
| S100A10 | 5 | 1.33 | 27 | 32 | 59 | 15 | 14 | 4 |
| | 20 | 0.71 | 108 | 35 | 143 | 51 | 40 | 20 |

Then, the hybridomas were colonized and the size of the colonies were visually identified. These colonies were cloned according to the above method and the subsequent growth was confirmed. For the supernatants of growing clones, the number of clones producing anti-S100A10 IgG1 antibody was identified by S100A10 ELISA. As described in the above Table 1, it was confirmed that IgG1 can be prepared by the in vitro immunization. The number of days required for the immunization step was about 5 days, which was a very short time for preparing IgG1 against the S100A10 protein compared to the conventional process (2-3 months).

### Example 2

The present example describes the step of extracting the anti-S100A10 antibody gene and expressing antibody in Escherichia coli.

### DNA extraction, and preparation of a single chain antibody scFv plasmid

From the B cells and hybridomas 1.5 × 10⁶ cells after immunization, total RNA was obtained using Isogen (manufactured by NIPPON GENE). The immunized cells obtained in the in vitro immunization step described in the above Example 1 may also be used in this step.

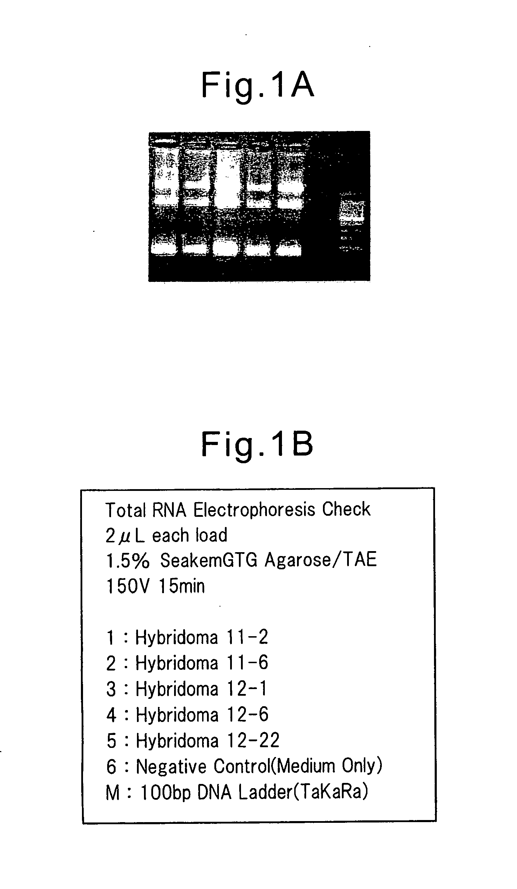

Then, the acquisition of total RNA was confirmed by 1.2% agarose gel electrophoresis. The results obtained are shown in Figure 1A and Figure 1B.

Using the total RNA obtained as the template and the mouse VH gene-specific primer and the mouse VL gene-specific primer as the primer, a single chain cDNA was synthesized using the ReverTra Ace (TOYOBO) reagent. The 1st PCR was carried out with the cDNA prepared as the template, and a primer VH forward primer mix/VH reverse primer mix or forward primer mix/VL reverse primer mix using the PrimeSTAR HS Polymerase (manufactured by TaKaRa). The primer sequence used has been introduced in Antibody Engineering: a practical approach (pp. 210-211).

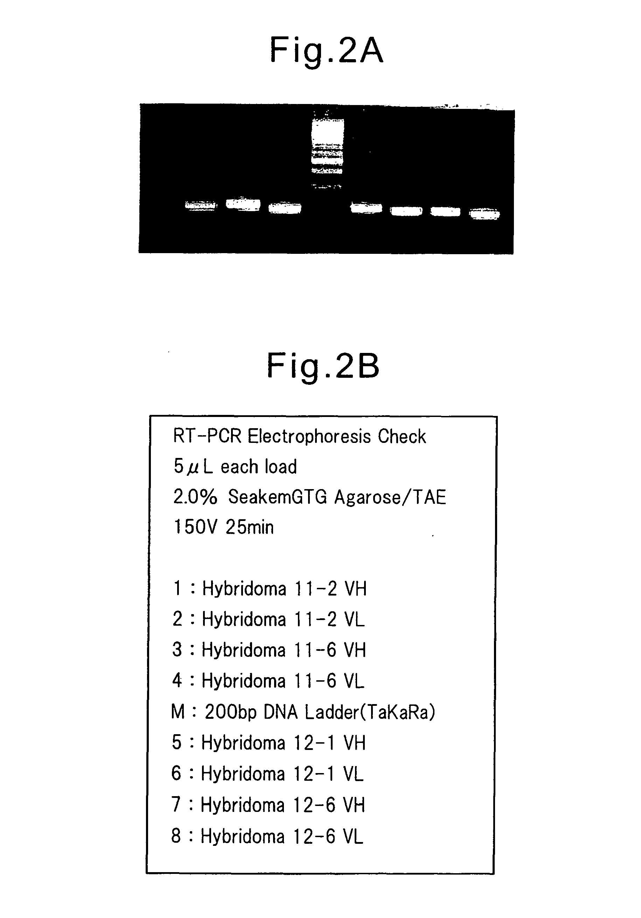

Then, its acquisition was confirmed by 2% agarose gel electrophoresis. The results obtained are shown in Figure 2A and Figure 2B.

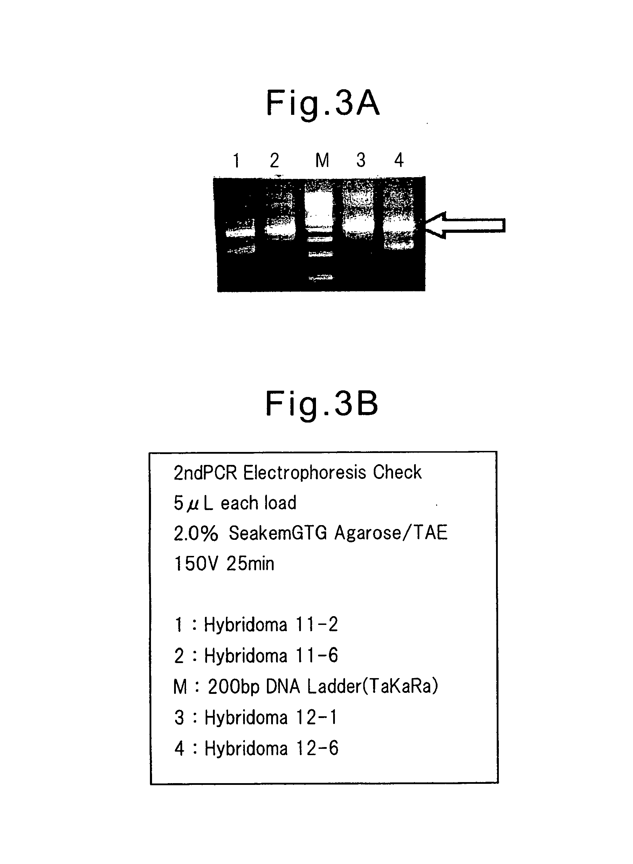

From the agarose gel after electrophoresis, the band of interest was excised, and the VH gene and VL gene were extracted using the QIAquick Gel Extraction kit (manufactured by QIAGEN) and purified. Subsequently, using 10 ng each of the VH gene and the VL gene, Link PCR was carried out using the PrimeSTAR HS Polymerase (manufactured by TaKaRa) without adding a primer. Then, a primer 2nd PCR Primer Premix was added to the sample of Link PCR, and subjected as it is to PCR amplification. Subsequently, using 2% agarose gel electrophoresis, its acquisition was confirmed. The results obtained are shown in Figure 3A and Figure 3B. These results confirmed that the desired scFv PCR product was obtained.

Subsequently, the scFv PCR product was digested with restriction enzymes EcoRI and HindIII at 37°C for 1 hour, and from the agarose gel after electrophoresis, the band of interest was excised, and using the QIAquick Gel Extraction kit (manufactured by QIAGEN) the scFv gene was extracted and puriried. Similarly, a plasmid vector pMAL-p2X was digested with EcoRI and HindIII at 37°C for 1 hour, and from the agarose gel after electrophoresis, the band of interest was excised, and using the QIAquick Gel Extraction kit (manufactured by QIAGEN) a plasmid vector gene was extracted and puriried. These purified products were mixed with the plasmid vector gene at a ratio of 6 mole of the scFv gene relative to 1 mol of the plasmid vector gene, and ligated using the DNA Ligation kit <Mighty Mix> (manufactured by TaKaRa). The ligation solution (20 µl) was mixed with the competent cells of Escherichia coli strain JM109 to transform the Escherichia coli strain JM109. The transformant was plated on a LB agar medium containing 50 µg/mL ampicillin and cultured at 37°C for 18 hours.

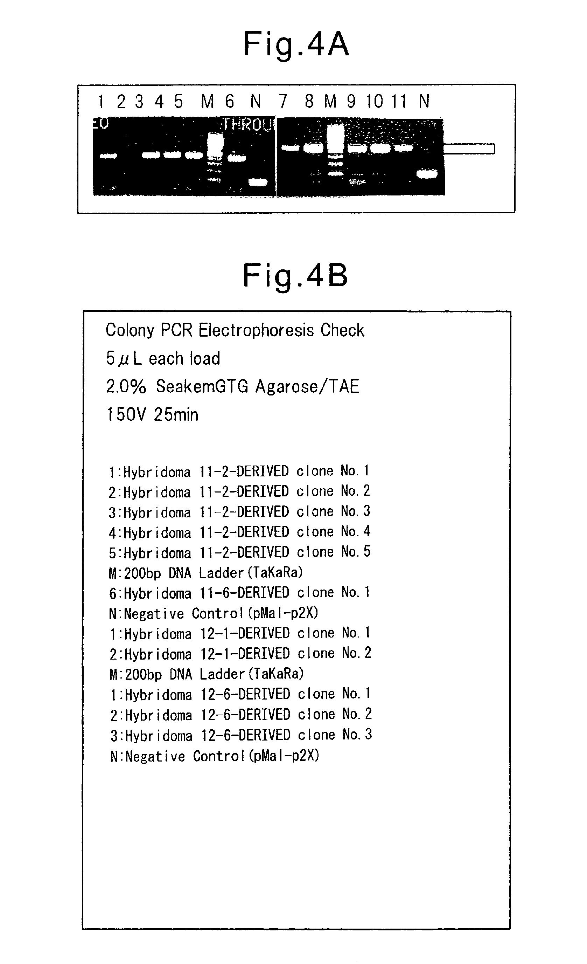

In order to select Escherichia coli clones having a plasmid ligated to the scFv gene, colonies formed in the above process were subjected to the colony PCR method, and by a 2% agarose gel electrophoresis, acquisition of them was confirmed. The results obtained are shown in Figure 4A and Figure 4B.

Clones obtained as above were cultured in a LB agar medium containing 50 µg/mL ampicillin and cultured at 37°C for 18 hours, and using the QIAquick Miniprep kit (manufactured by QIAGEN) plasmid DNA was extracted. The DNA sequence of the plasmid extracted was identified using the DNA Sequencer MegaBASE 1000 (manufactured by GE Healthcare). It took three days to obtain this antibody gene.

### Expression of a single chain antibody scFv by Escherichia coli

Using a plasmid of which sequence has been identified, a Escherichia coli strain BL21(DE3) was transformed. The transformant was plated on a LB agar medium containing 50 µg/mL ampicillin and cultured at 37°C for 18 hours.

Subsequently, the colonies formed as above were cultured for 18 hours in 5 ml of a LBG liquid medium containing 50 µg/mL ampicillin. One mL of this culture liquid was cultured at 30°C in 100 ml of a LBG liquid medium containing 50 µg/mL ampicillin. For every hour of culture, absorbance at 660 nm was measured with a spectrophotometer UV-1200 (manufactured by Shimadzu Corporation). When the absorbance reached 0.5, IPTG was added to a final concentration of 0.5 mM and cultured for 20 hours at 23°C. After the completion of culturing, it was centrifuged (6000 rpm, 20 min, 4°C) and the cells of Escherichia coli were collected. They were dissolved in 5 mL of Sol. A (20% sucrose, 1 mM EDTA, 30 mM Tris-HCl, pH8.0, 0.5 mM APMSF), and allowed to stand at room temperature for 10 minutes. By centrifuging (6000 rpm, 20 min, 4°C), the cells of Escherichia coli were collected, dissolved in 5 mL of ice-cold Sol. B (5 mM MgSO₄), and allowed to stand on ice for 10 minutes. Then, by centrifuging (6000 rpm, 20 min, 4°C), the supernatant was collected.

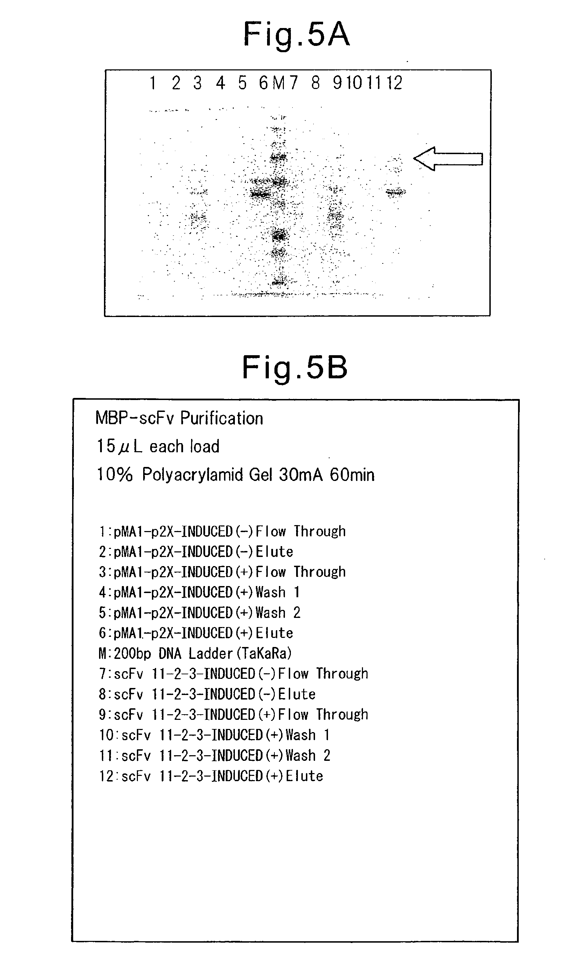

Subsequently, the supernatant collected as above was subjected to an amylose resin column (10 mL, ϕ2.5 cm × 3.3 cm). After the entire supernatant has passed through the column, the column was washed in 40 mL of a washing buffer (200 mM NaCl, 20 mM Tris-HCl, pH8.0). After conducting this washing step twice, the MBP (maltose binding protein)-scFv protein adsorbed to the column was eluted with 40 mL of an Eluting buffer (5 mM maltose, 200 mM NaCl, 20 mM Tris-HCl, pH8.0), and purified. The purified MBP-scFv protein was analyzed by a SDS-PAGE method. The results obtained are shown in Figure 5A and Figure 5B.

Also, the concentration of the purified MBP-scFv protein was determined using an assay kit (Dc Protein Assay kit, manufactured by BioRad). The result revealed that 0.345 mg of the MBP-scFv protein was obtained from 50 mL of the culture liquid.

Incidentally, the above purification procedure took three days. When counted from the step of obtaining the antibody gene, the present method is over in six days, indicating that scFv to the S100A10 protein was obtained in a very short period of time compared to the conventional method (requiring 2-3 months).

Though it was shown in the example that the present invention makes it possible to produce anti-S100A10 antibody in large quantities, it should be noted that the antibody is not limited to the anti-S100A10 antibody and may advantageously be applied to various other antibodies.

### Example 3

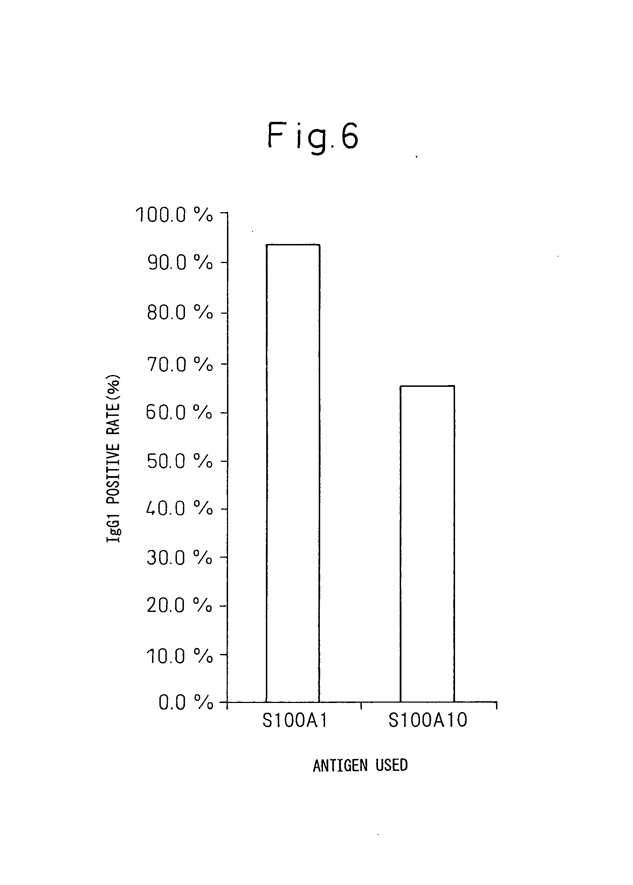

The procedure described in the above Example 1 was repeated. In this example, however, the S10A1 protein and the S100A10 protein were used as antigens and the in vitro immunization was conducted in a manner similar to the above Example 1, and IgG1 positivity was calculated. Figure 6 plots the result thus obtained. Though there is some variation depending on the antigen, it can be confirmed from Figure 6, that class switching to IgG1 has occurred in both cases at high rates of not smaller than 60%.

In accordance with the present invention, it was demonstrated that in the in vitro immunization, an effective class switching can be attained by adding a stimulating substance such as LPS (40 µg/mL), IL-4, IL-5 (10 ng/mL), anti-CD38 antibody and anti-CD40 antibody (1 µg/mL) once after immunization, and culturing for four days in the presence of the stimulating substance.

### Example 4

The procedure described in the above Example 1 was repeated. However in this example, the S100A10 protein were used as the antigen, the in vitro immunization was conducted in a manner similar to the above Example 1, and the entire cells were collected on day 4 and day 7 of culture, from which total RNA was obtained.

With 600 ng of total RNA as the template, a reverse transcription reaction was conducted using the ReverTra Ace (TOYOBO). The reaction composition was a volume of 20 µl according to the manufacturer's protocol, and as the primer, The Oligo(dT)20 (TOYOBO) was used. The reaction condition was 42°C for 20 minutes, 99°C for 5 minutes, and 4°C for 5 minutes. With 1 µl of this product as the template, real-time PCR was conducted using the PowerSYBR Green PCR Master Mix (Applied Biosystems). The reaction condition comprised 95°C for 10 minutes to 40 cycles of 95°C for 15 seconds and 60°C for 1 minute.

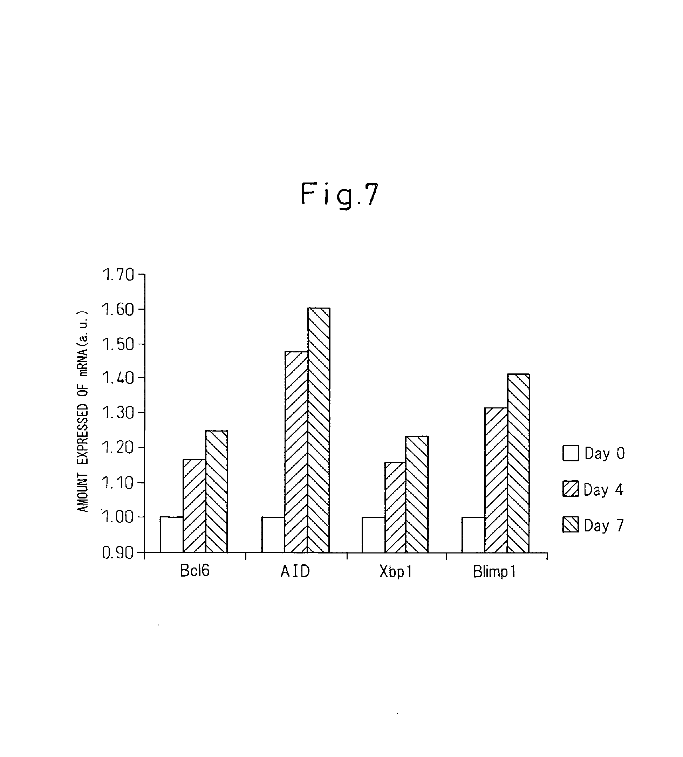

The equipment used in this example is the 7500Fast (Applied Biosystems). The result obtained was normalized using an internal standard, β actin, and made into a graph. The result obtained is shown in Figure 7. It can be seen from Figure 7 that the enhanced expression of all the markers (Bcl6, AID, Xbp1, Blimp1) was identified, and the affinity of antibodies and the induction of differentiation of antibody-producing cells are further increased. As used herein, Bcl6 and AID are genes involved in the affinity maturation and class switching of antibody, and Xbp1 is a multi-functional transcription factor which is also implicated in the differentiation into antibody-producing cells, and Blimp1 is also a factor acting in the differentiation of B cells into antibody-producing cells (plasma cells) as Xbp1 is.

### Example 5

The present example describes an experimental method of cytotoxicity testing of cytokines.

BALB/c mice (4 week-old, female, SPF/VAF) were sacrificed by cervical dislocation. After visual identification, the spleens of the mice were removed and washed in 70% ethanol. Furthermore, after washing in PBS, the spleens from two animals were placed on a cell strainer, and mashed with a cell scraper. By washing the cell strainer with 4 mL of PBS(-) for three times, spleen cells were eluted from the cell strainer. They were centrifuged (1300 rpm × 3 min, 4°C) and the cells were collected.

After suspending the cells in 3 mL of the ACK lysing buffer, 10 mL of PBS(-) was added to remove erhythrocytes, centrifuged (1300 rpm × 3 min, 4°C) and the cells were collected. The spleen cells were suspended in 10 mL of the RPMI1640 medium, and by passing them through a cell strainer, insoluble fats, etc., were removed. The number of cells was counted using a hematocytometer and then concentrated to 1 × 10⁷ cells/mL.

Stimulating substances were prepared at 10,000 times the normal final concentrations (the normal final concentration of IL-4 is 10 ng/mL, the normal final concentration of IL-5 is 10 ng/mL, the normal final concentration of anti-CD38 antibody is 1 µg/mL, the normal final concentration of anti-CD40 antibody is 1 µg/mL, the normal final concentration of LPS is 40 µg/mL). They were serially ten-fold diluted using PBS(-). By so doing, dilution series of stimulating substances were prepared to 10⁻⁶ to 10⁴-fold. 1.5 µl (1/1000 the volume) of a stimulating substance was mixed with 300 µl of spleen cells, and adjusted to a cell concentration of 2 × 10⁶ cells/mL with a RPMI1640 medium containing 40% FBS, and cultured for 4 days at 37°C in a CO₂ incubator.

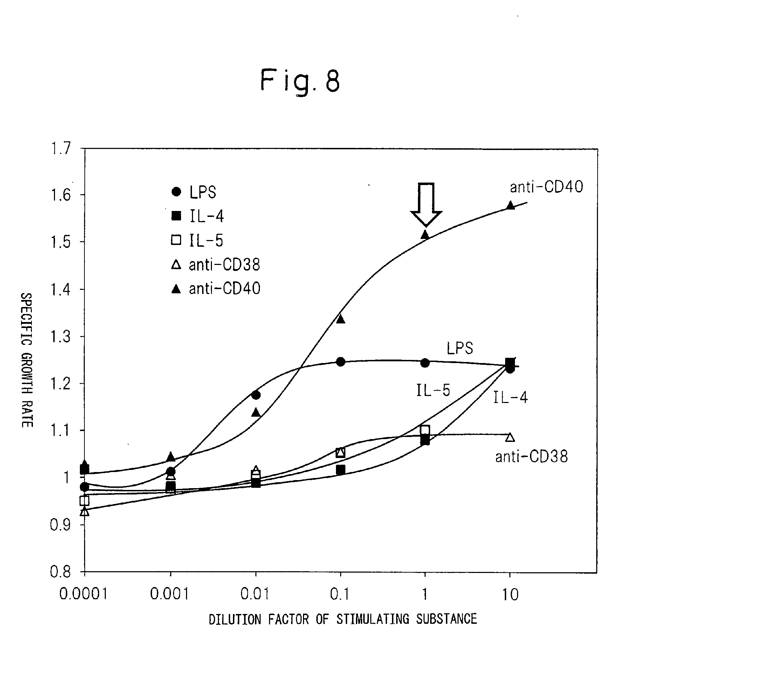

After culturing, 100 µl of the cell solution was aliquoted into a 96-well plate and the temperature was maintained for 2 hours in a 37°C CO₂ incubator. Ten mL of the Cell Counting kit-8 (manufactured by Dojindo) was added thereto, and the temperature was maintained for 1 more hour. Using a microplate reader, absorbance at 450 nm was measured, and as the blank, absorbance at 650 nm was measured. By subtracting the measured value at 650nm from the measured value at 450 nm, the measured value was obtained. By dividing each measured value by the measured value without addition of a stimulating substance, specific growth rate was obtained. The results obtained are shown in the graph in Figure 8. When creating a graph, results not greater than 10⁻⁵ exhibited the same results of 10⁻⁴ , and thus were omitted. The final concentrations of stimulating substances at each dilution factor are as shown in the following Table 2.

**Table 2**

| Dilution factor | LPS | IL-4 | IL-5 | Anti-CD38 antibody | Anti-CD40 antibody |
|---|---|---|---|---|---|
| 10 | 400µg/mL | 100ng/mL | 100ng/mL | 10µg/mL | 10µg/mL |
| 1 | 40µg/mL | 10ng/mL | 10ng/mL | 1µg/mL | µg/mL |
| 0.1 | 4µg/mL | 1ng/mL | 1ng/mL | 100ng/mL | 100ng/mL |
| 0.01 | 400ng/mL | 100pg/mL | 100pg/mL | 10ng/mL | 10ng/mL |
| 0.001 | 40ng/mL | 10pg/mL | 10pg/mL | 1ng/mL | 1ng/mL |
| 0.0001 | 4ng/mL | 1pg/mL | 1pg/mL | 100pg/mL | 100pg/mL |
| 10⁻⁵ | 400pg/mL | 100fg/mL | 100fg/mL | 10pg/mL | 10pg/mL |
| 10⁻⁶ | 40pg/mL | 10fg/mL | 10fg/mL | 1pg/mL | 1pg/mL |
| 10⁻⁷ | 4pg/mL | 1fg/mL | 1fg/mL | 100fg/mL | 100fg/mL |
| 10⁻⁸ | 400fg/mL | 100at/mL | 100at/mL | 10fg/mL | 10fg/mL |
| 10⁻⁹ | 40fg/mL | 10ag/mL | 10ag/mL | 1fg/mL | 1fg/mL |

As described above, the result of cytotoxicity testing of cytokines is shown in the graph of Figure 8, in which the ordinate represents the specific growth rate and the abscissa represents the dilution factor of the stimulating substance. In the figure, the concentration indicated by an arrow is a concentration normally used. The experiment was conducted independently for three times and the mean is shown in the graph.

Cytotoxicity was not observed at the normal final concentrations. For LPS, cell death was observed sometimes by using the 10-fold concentration of the current concentration. However the reduction was at the same extent as when no stimulating substance was added, and thus it was demonstrated that the concentration can be used. Also, cellular growth was observed for the addition of any stimulating substance at the normal final concentration, it may be thought that they are efficiently immunized.

### Example 6

The present example describes an experimental method of investigating combinations of stimulating substances.

BALB/c mice (4 week-old, female, SPF/VAF) were sacrificed by cervical dislocation. After visual identification, the spleens of the mice were removed and washed in 70% ethanol. Furthermore, after washing in PBS, the spleens from two animals were placed on a cell strainer, and mashed with a cell scraper. By washing the cell strainer with 4 mL of PBS(-) for three times, spleen cells were eluted from the cell strainer. They were centrifuged (1300 rpm × 3 min, 4°C) and the cells were collected.

After suspending the cells in 3 mL of the ACK lysing buffer, 10 mL of PBS(-) was added to remove erhythrocytes, centrifuged (1300 rpm × 3 min, 4°C) and the cells were collected. The spleen cells were suspended in 10 mL of the RPMI1640 medium, and by passing them through a cell strainer, insoluble fats, etc., were removed. The cells were counted using a hematocytometer and then concentrated to 1 × 10⁷ cells/mL.

Stimulating substances were prepared at 1000 times the normal final concentrations (the normal final concentration of IL-4 is 10 ng/mL, the normal final concentration of IL-5 is 10 ng/mL, the normal final concentration of anti-CD38 antibody is 1 µg/mL, the normal final concentration of anti-CD40 antibody is 1 µg/mL, the normal final concentration of LPS is 40 µg/mL). 1.5 µl (1/1000 the volume) of a stimulating substance was mixed with 300 µl of spleen cells, and adjusted to a cell concentration of 2 × 10⁶ cells/mL with a RPMI1640 medium containing 40% FBS, and cultured for 4 days at 37°C in a CO₂ incubator.

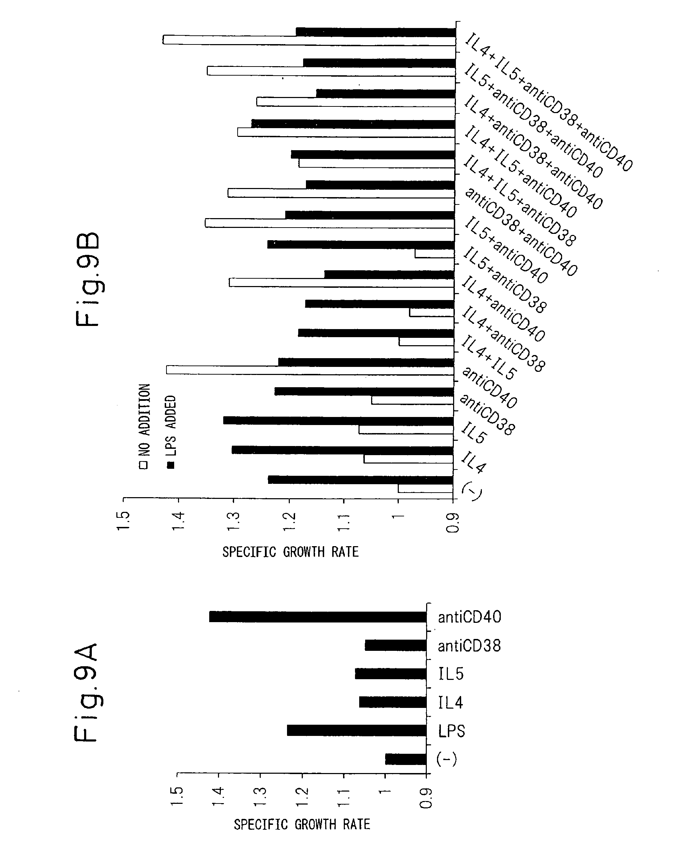

After culturing, 100 µl of the cell solution was aliquoted into a 96-well plate and the temperature was maintained for 2 hours in a 37°C CO₂ incubator. Ten mL of the Cell Counting kit-8 (manufactured by Dojindo) was added thereto, and the temperature was maintained for 1 more hour. Using a microplate reader, absorbance at 450 nm was measured, and as the blank, absorbance at 650 nm was measured. By subtracting the measured value at 650nm from the measured value at 450 nm, the measured value was obtained. By dividing each measured value by the measured value without addition of a stimulating substance, specific growth rate was obtained. The results obtained are shown in Figure 9A and Figure 9B. The ordinate represents specific growth rate and the abscissa represents the type of a stimulating substance.

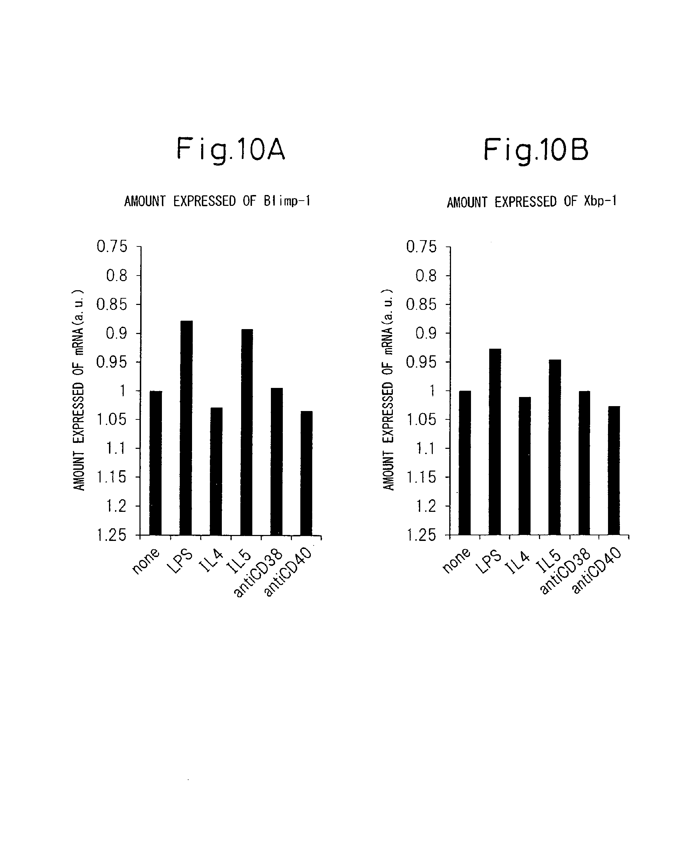

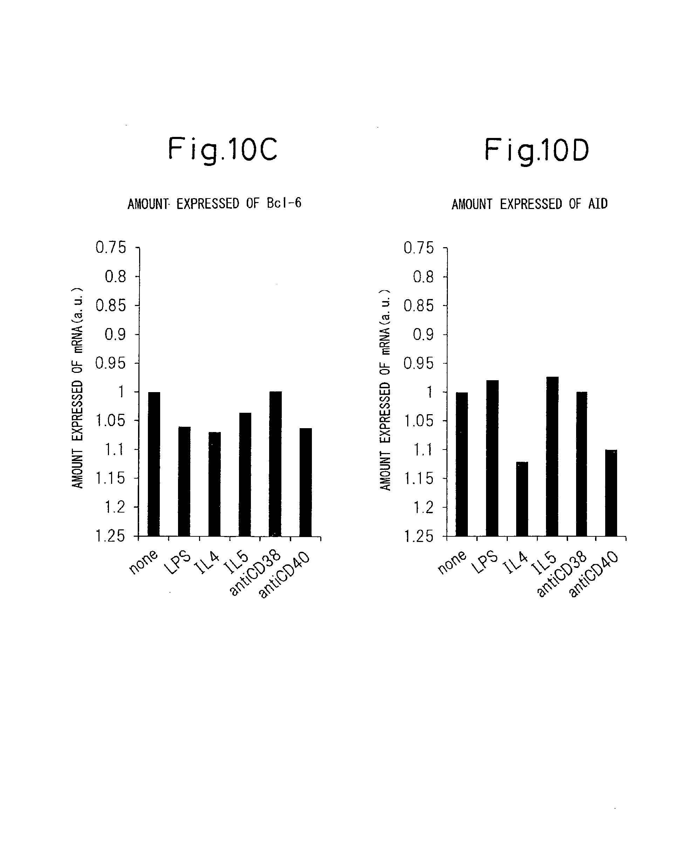

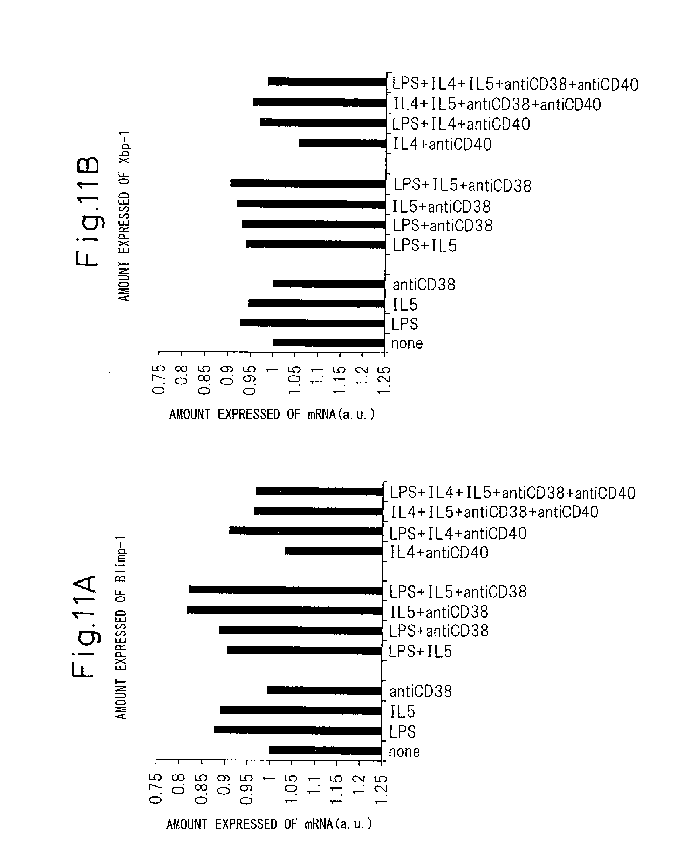

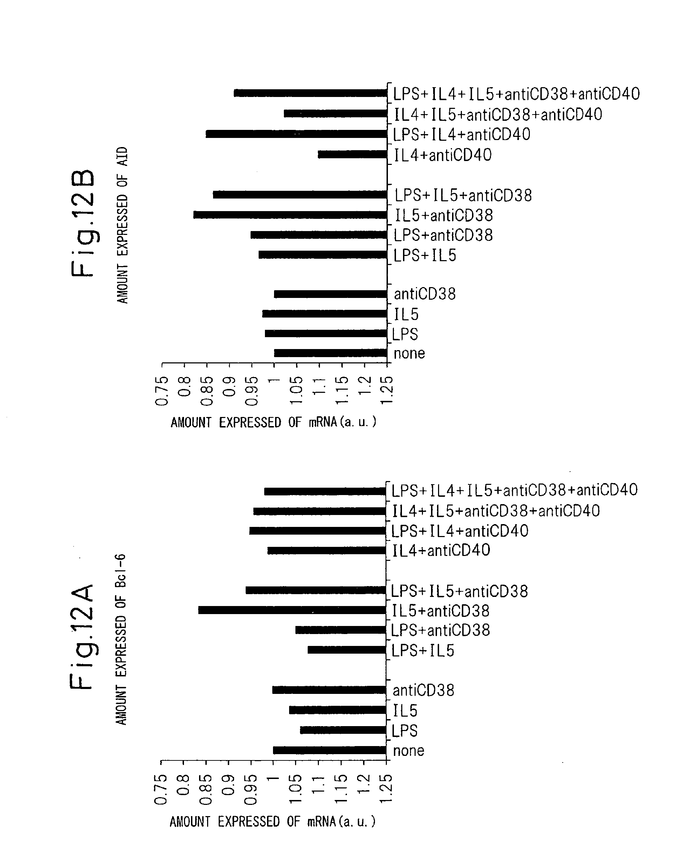

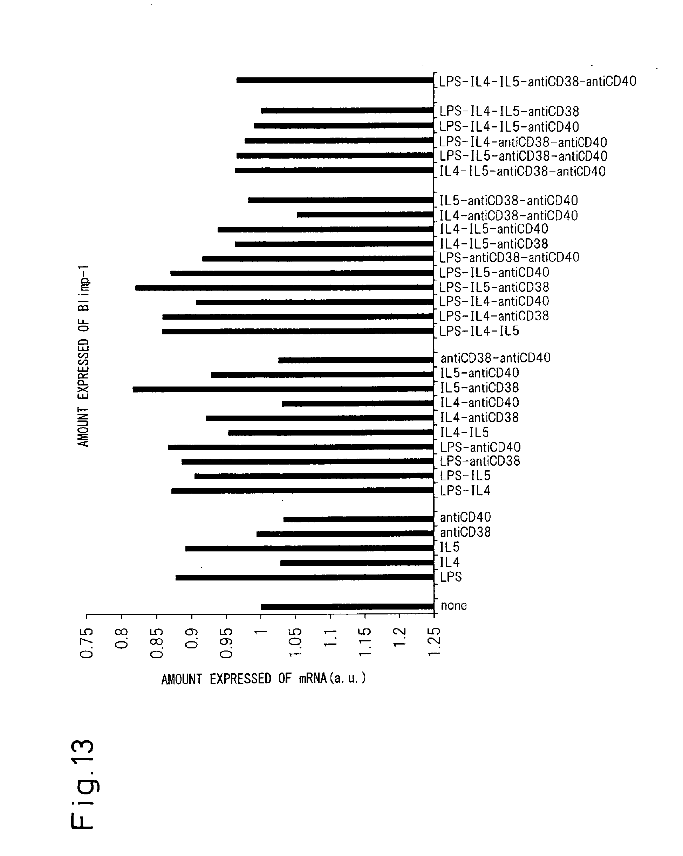

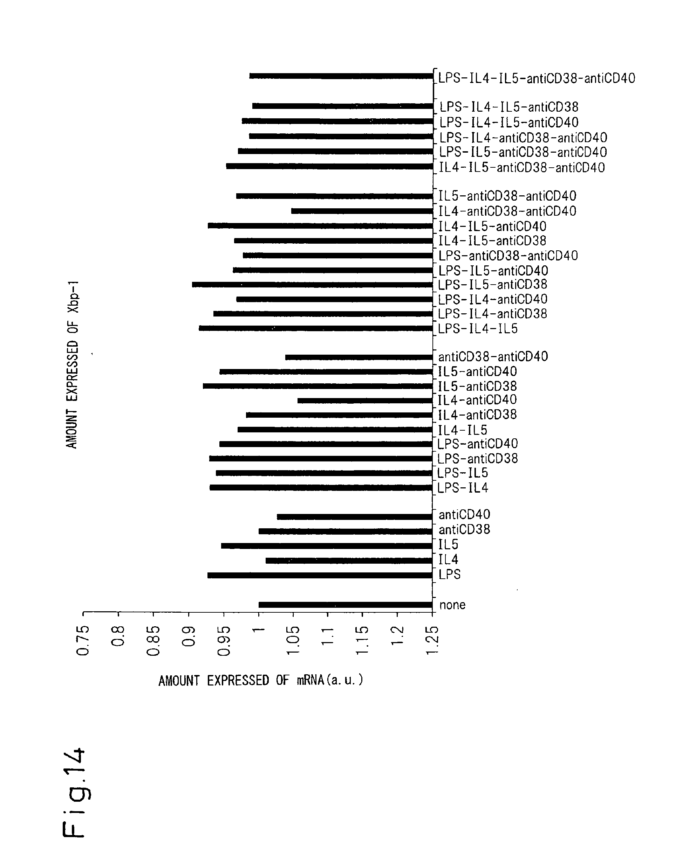

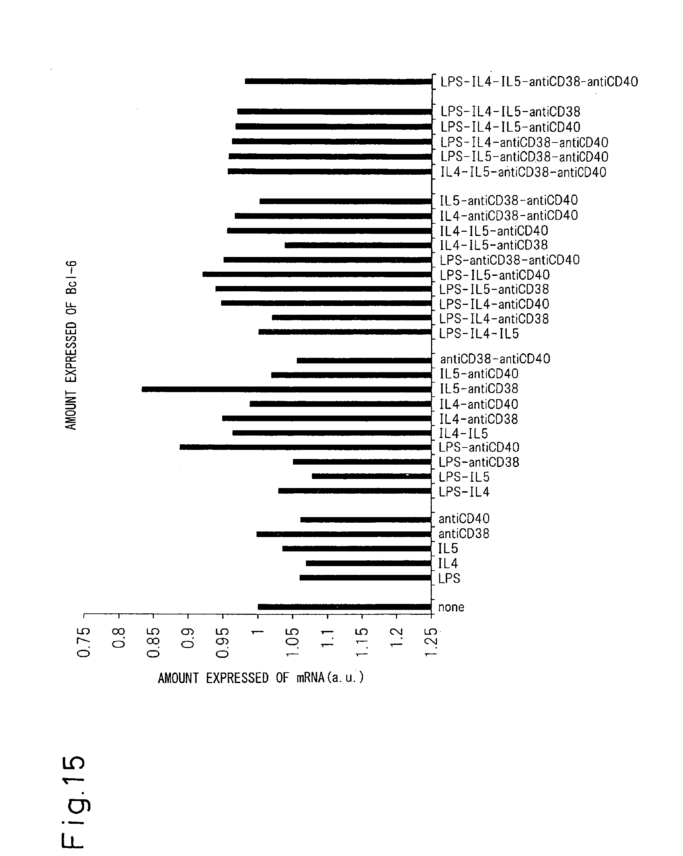

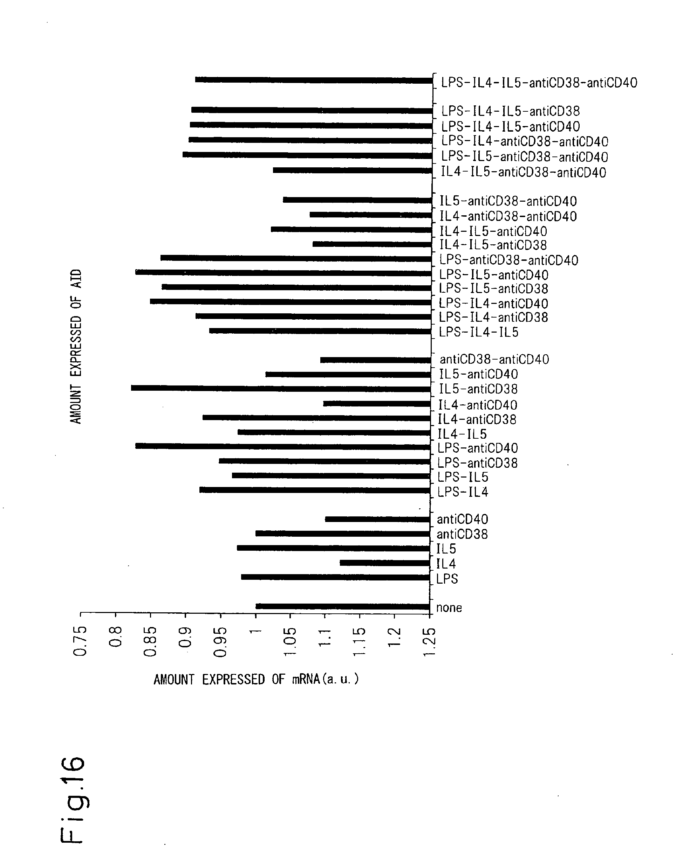

The rest of the cells after culturing were collected, and using ISOGEN (manufactured by Nippon Gene) total RNA was collected. With 600 ng of this total RNA as the template, a reverse transcription reaction was conducted using the ReverTra Ace (TOYOBO). The reaction composition was a volume of 20 µl according to the manufacturer's protocol, and as the primer, The Oligo(dT)20 (TOYOBO) was used. The reaction condition was 42°C for 20 minutes, 99°C for 5 minutes, and 4°C for 5 minutes. With 1 µl of this product as the template, real-time PCR was conducted using the PowerSYBR Green PCR Master Mix (Applied Biosystems). The reaction condition comprised 95°C for 10 minutes to 40 cycles of 95°C for 15 seconds and 60°C for 1 minute. The equipment used in this example is the 7500Fast (Applied Biosystems). The result obtained was normalized using an internal standard, β actin, and made into graphs. The numerical value obtained was further normalized using the value at no addition. The results obtained are shown in Figure 10A to Figure 10D, Figure 11A and Figure 11B, Figure 12A and Figure 12B, Figure 13, Figure 14, Figure 15 and Figure 16.

It can be seen from a series of graphs showing the results of investigation on the combination of stimulating substances, it was revealed that for cell growth, stimulation with anti-CD40 and with LPS are effective. However, it was found that when LPS and anti-CD40 coexist, growth was suppressed. It is believed that this is because apoptosis was induced since the stimulation to the cells was too strong.

On the other hand, the amounts expressed of differentiation markers for plasma cells were increased compared to when either of LPS, anti-CD38 and IL5 was added, suggesting that these stimulations cause differentiation to progress. Also, by combined addition of these stimulations, enhancement in the amount expressed was confirmed. Since LPS has an effect of stimulating cellular growth, results were obtained that its coexistence enhanced the total number of cells with a result that the amount expressed decreased. Also, since the amounts expressed of differentiation markers are almost saturated by adding any two of them, it was revealed that the stimulation of differentiation fully serves it purpose with the current concentration used. Furthermore, it was confirmed that though the addition of IL4 and anti-CD40 increased the total number of cells, resulting in the decreased amount expressed, the amounts expressed of differentiation markers can be enhanced by adding thereto IL5, anti-CD38 and LPS.

Furthermore, the amounts expressed of markers of somatic mutation were similarly increased when either of LPS, anti-CD38 and IL5 was added. Under a condition in which cells do not grow well (condition in which IL4, anti-CD40 or LPS is not present), the amounts expressed of markers of somatic mutation are enhanced by stimulation with IL5 and anti-CD38, but under a condition in which cells grow, the amounts expressed of markers were confirmed to be enhanced by LPS stimulation. This suggests that the activation and differentiation of B cells progress in an antigen-dependent manner by LPS. LPS is a lipopolysaccharide constituting the peptideglycan of Escherichia coli, and when this coexists, it indicates a state of being infected with microorganisms. From the viewpoint of biological defense, immunocompetent cells are equipped with an ability of inducing the somatic mutation and activation of B cells and producing more suitable antibodies in response to LPS stimulation.

It was expected in this in vitro immunization that among the B cells originally present in the spleen cells, cells producing the antibody of interest may grow by IL4 and anti-CD40, undergo somatic mutation by IL5 and anti-CD38, and turn to cell groups having a variety of affinities. This was suggested to be a system in which cells that do not react with antigen grow and undergo somatic mutation by LPS stimulation and accumulate mutations so as to be able to react with antigen.

### Example 7

The present example describes an experimental method of investigating the amounts expressed of markers at the upper and lower limit of cytokine concentrations.

BALB/c mice (4 week-old, female, SPF/VAF) were sacrificed by cervical dislocation. After visual identification, the spleens of the mice were removed and washed in 70% ethanol. Furthermore, after washing in PBS, the spleens from two animals were placed on a cell strainer, and mashed with a cell scraper. By washing the cell strainer with 4 mL of PBS(-) for three times, spleen cells were eluted from the cell strainer. They were centrifuged (1300 rpm × 3 min, 4°C) and the cells were collected.

After suspending the cells in 3 mL of the ACK lysing buffer, 10 mL of PBS(-) was added to remove erhythrocytes, centrifuged (1300 rpm × 3 min, 4°C) and the spleen cells were collected. The spleen cells were suspended in 10 mL of the RPMI1640 medium, and by passing them through a cell strainer, insoluble fats etc. were removed. The number of cells was counted using a hematocytometer and then concentrated to 1 × 10⁷ cells/mL.

With the hS100A10 protein at 0.1 nmol or 1 nmol as the antigen, 1 × 10⁷ spleen cells were immunized. At this time, 50 µg of muramyl dipeptide (SIGMA) was added as an adjuvant. The immune reaction was conducted by allowing to stand at room temperature for 15 minutes. The stimulating substances (IL-4, IL-5, anti-CD38 antibody, anti-CD40 antibody, LPS) were prepared at final concentrations shown in the following Table 3. The stimulating substance and the immunized spleen cells were mixed. The cell concentration was adjusted to 2 × 10⁶ cells/mL with a RPMI1640 medium containing 40% FBS, and cultured for four days in a 37°C CO₂ incubator.

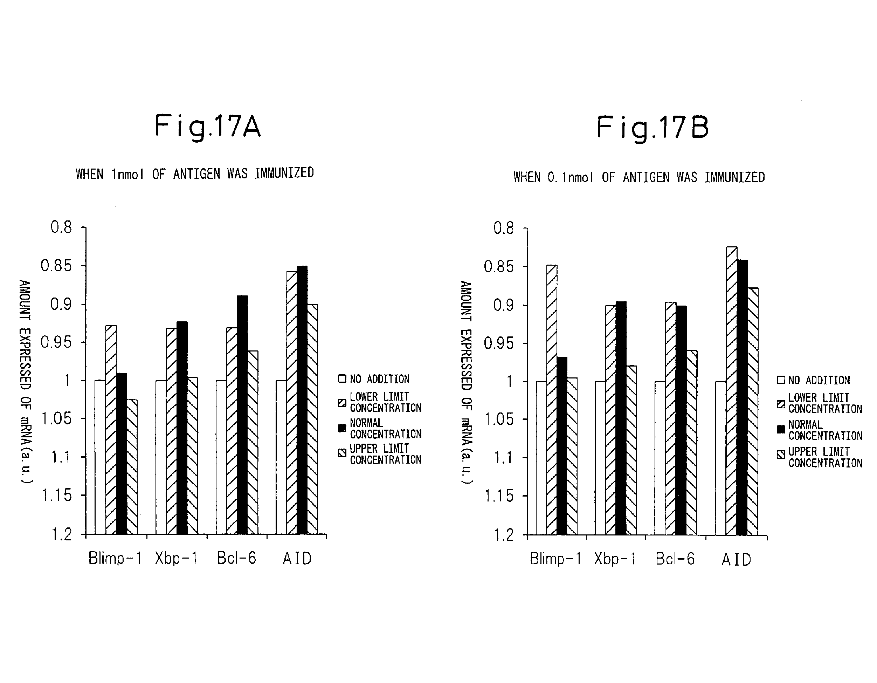

After culturing, all the cells were collected, and using ISOGEN (manufactured by Nippon Gene) total RNA was collected. With 600 ng of this total RNA as the template, a reverse transcription reaction was conducted using the ReverTra Ace (TOYOBO). The reaction composition was a volume of 20 µl according to the manufacturer's protocol, and as the primer, The Oligo(dT)20 (TOYOBO) was used. The reaction condition was 42°C for 20 minutes, 99°C for 5 minutes, and 4°C for 5 minutes. With 1 µl of this product as the template, real-time PCR was conducted using the PowerSYBR Green PCR Master Mix (Applied Biosystems). The reaction condition comprised 95°C for 10 minutes to 40 cycles of 95°C for 15 seconds and 60°C for 1 minute. The equipment used in this example is the 7500Fast (Applied Biosystems). The result obtained was normalized using an internal standard, β actin. The numerical value obtained was further normalized using the value at no addition and made into graphs. The results obtained are shown in Figure 17A and Figure 17B.

**Table 3**

| Dilution factor | LPS | IL-4 | IL-5 | anti-CD38 antibody | anti-CD40 antibody |
|---|---|---|---|---|---|
| No addition | 0µg/mL | 0ng/mL | 0ng/mL | 0µg/mL, | 0µg/mL |
| Lower limit concentration | 10ng/mL | 1pg/mL | 1pg/mL | 1ng/mL | 1ng/mL |
| Normal concentration | 40µg/mL | 10ng/mL | 10ng/mL | 1µg/mL | 1µg/mL |
| Upper limit concentration | 500µg/mL | 50ng/mL | 50ng/mL | 50µg/mL | 50µg/mL |

In Figure 17A and Figure 17B showing the results on the amounts expressed of markers at the upper and lower limit concentrations of cytokines, the abscissa of the graph represents the name of the marker investigated and the ordinate represents the amount expressed of mRNA. In the ordinate of the graph, when the value decreased by 0.05, it can be converted to about twice the amount expressed of mRNA.

From the figures, whether the antigen concentration is 0.1 nmol or 1 nmol, the expression of markers equal to or greater than that at no addition was confirmed at the upper and lower limit concentrations of stimulating substances. This suggested that by adding a stimulating substance in this range, the antigen-immunized cells differentiate into plasma cells, and in the process the affinity maturation and class switching of antibody occurs.

### Example 8

The present example describes an experimental method of investigating B cell differentiation.

BALB/c mice (4 week-old, female, SPF/VAF) were sacrificed by cervical dislocation. After visual identification, the spleens of the mice were removed and washed in 70% ethanol. Furthermore, after washing in PBS, the spleens from two animals were placed on a cell strainer, and mashed with a cell scraper. By washing the cell strainer with 4 mL of PBS(-) for three times, spleen cells were eluted from the cell strainer. They were centrifuged (1300 rpm × 3 min, 4°C) and the cells were collected.

After suspending the cells in 3 mL of the ACK lysing buffer, 10 mL of PBS(-) was added to remove erhythrocytes, centrifuged (1300 rpm × 3 min, 4°C) and the cells were collected. The spleen cells were suspended in 10 mL of the RPMI1640 medium, and by passing them through a cell strainer, insoluble fats, etc., were removed. The number of cells was counted using a hematocytometer and then concentrated to 1 × 10⁷ cells/mL.

With the purified hS100A10 protein as the antigen, 2 × 10⁷ spleen cells were immunized. At this time, 100 µg of muramyl dipeptide (SIGMA) was added as an adjuvant. The immune reaction was conducted by allowing to stand at room temperature for 15 minutes. The stimulating substances (IL-4, IL-5, anti-CD38 antibody, anti-CD40 antibody, LPS) were prepared at 1000 times that of the normal final concentrations. Ten µl of this stimulating substance and the immunized spleen cells (2 mL) were mixed. The cell concentration was adjusted to 2 × 10⁶ cells/mL with a RPMI1640 medium containing 40% FBS, and cultured for four days in a 37°C CO₂ incubator. As the control, the one in which the antigen was only added, the one in which the stimulating substance was only added, and the one in which none were added was prepared.

After culturing, all the cells were collected, and suspended in a staining buffer (a PBS(-) buffer containing 0.5% BSA and 2 mM EDTA) in order to attain 1 × 10⁸ cells/mL. Twenty µl of the cells was stained with antibody. The staining method is as shown in the following Table 4.

**Table 4**

| Target cell | Antibody | Staining method |
|---|---|---|
| B cells | anti-CD45R-PE (Miltenyi Biotec) | Twenty cl of antibody diluted 20-fold with the staining buffer was added. This was allowed to stand in a refrigerator for 10 minutes, and then the unreacted antibody was washed with the staining buffer. |
| Plasma cells | anti-Syndecan-1 (COSMO BIO) | Two µl of antibody was added. This was allowed to stand on ice for 45 minutes, and then the unreacted antibody was washed with a staining buffer. Subsequently, as the secondary antibody, anti-IgG(F(ab')₂), goat (donkey), PE-Cy5-labelled antibody was diluted 20-fold with the staining buffer, and two µl of it was added. This was allowed to stand on ice for 45 minutes, and then the unreacted antibody was washed with a staining buffer. |
| T1-B cells | anti-C1qR(M-20), Mouse(Goat) (a.k.a. anti-CD93) (COSMO BIO) anti-CD23-FITC (COSMO BIO) | Two µl each of antibodies was added. This was allowed to stand on ice for 45 minutes, and then the unreacted antibody was washed with a staining buffer. Subsequently, as the secondary antibody, anti-IgG(F(ab')₂), goat (donkey), PE-Cy5-labelled antibody was diluted 20-fold with the staining buffer, and two µl of it was added. This was allowed to stand on ice for 45 minutes, and then the unreacted antibody was washed with a staining buffer. |
| T2-B cells | anti-C1qR(M-20), Mouse(Goat) (a.k.a. anti-CD93) (COSMO BIO) anti-CD23-FITC (COSMO BIO) | Two µl each of antibodies was added. This was allowed to stand on ice for 45 minutes, and then the unreacted antibody was washed with a staining buffer. Subsequently, as the secondary antibody, anti-IgG(F(ab')₂), goat (donkey), PE-Cy5-labelled antibody was diluted 20-fold with the staining buffer, and two µl of it was added. This was allowed to stand on ice for 45 minutes, and then the unreacted antibody was washed with a staining buffer. |
| B2 cells | anti-C1qR(M-20), Mouse(Goat) (a.k.a. anti-CD93) (COSMO BIO) anti-CD23-FITC (COSMO BIO) | Two µl each of antibodies was added. This was allowed to stand on ice for 45 minutes, and then the unreacted antibody was washed with a staining buffer. Subsequently, as the secondary antibody, anti-IgG(F(ab')₂), goat (donkey), PE-Cy5-labelled antibody was diluted 20-fold with the staining buffer, and two µl of it was added. This was allowed to stand on ice for 45 minutes, and then the unreacted antibody was washed with a staining buffer. |
| Activated B cells | anti-BCMA,mouse(Rat) (COSMO BIO) | Two µg of mouse IgG (SIGMA) was added and allowed to stand at room temperature for 15 minutes. Two µl of antibody was added thereto. This was allowed to stand on ice for 45 minutes, and then the unreacted antibody was washed with a staining buffer. |

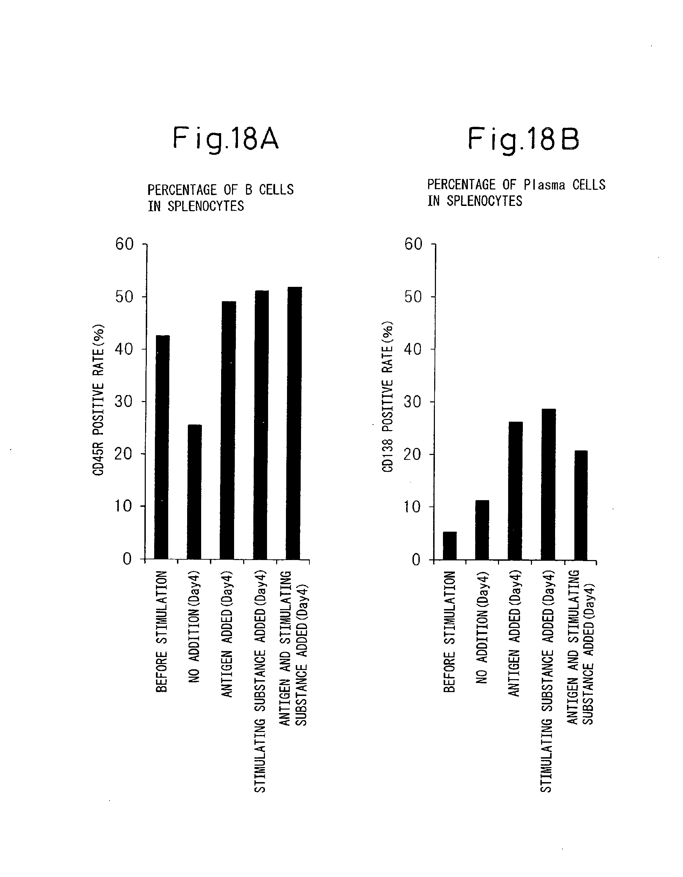

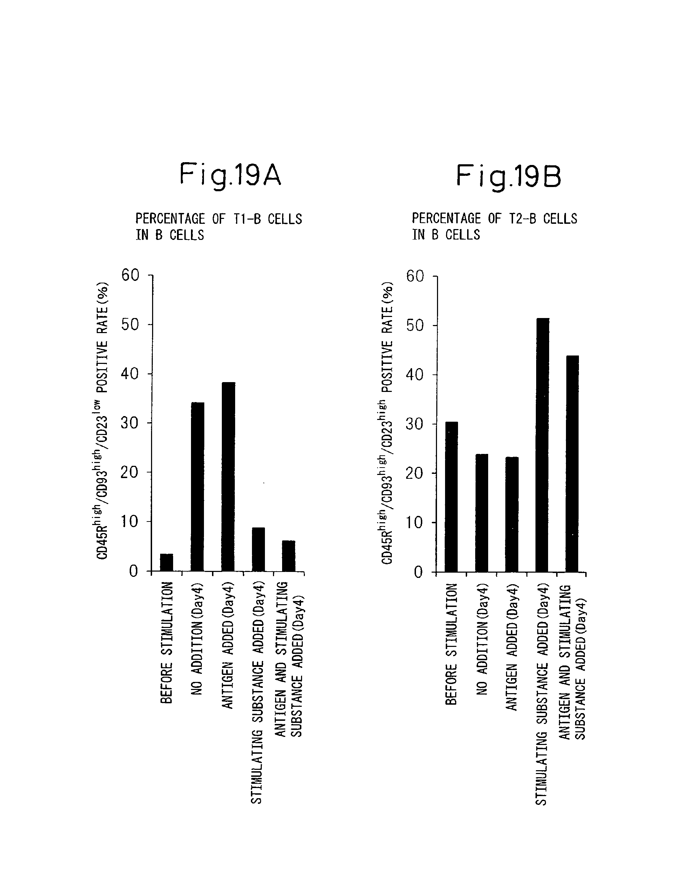

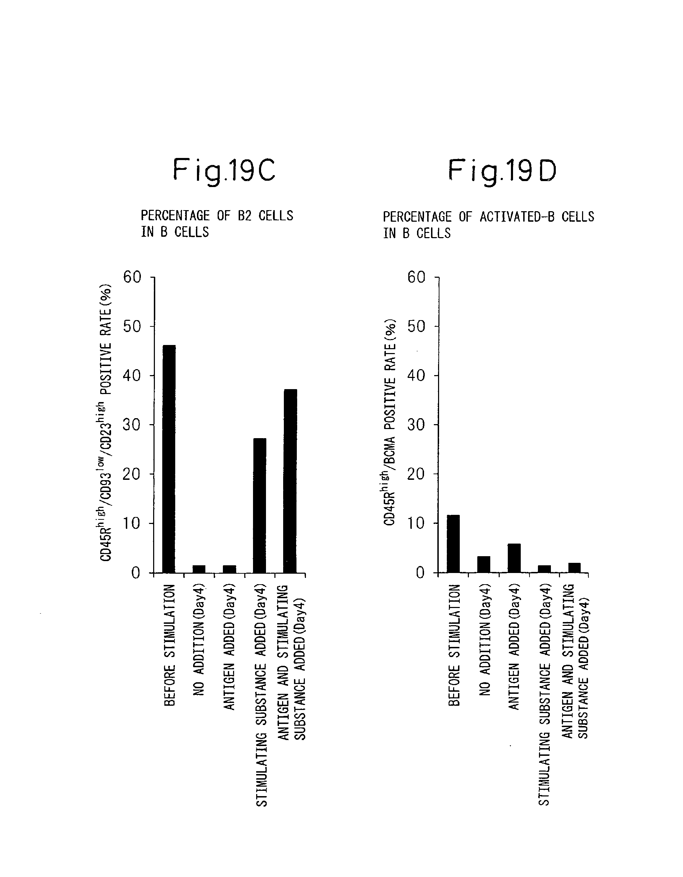

Stained cells were suspended in 500 mL of the staining buffer, and analyzed using FACSCaliber. Positivity of respective cells was determined and made into graphs. The results obtained are shown in Figure 18A, Figure 18B, and Figure 19A to Figure 19D. In the graph, the ordinate represents the positivity of respective cells, and the abscissa represents the presence or absence of antigen stimulation.

From a series of graphs indicating the results of investigation on B cell differentiation, it was confirmed that activation and propagation of B cells and differentiation into plasma cells can be effected by immunizing spleen cells in vitro. It was revealed that activation and maturation of B cells are not cause by antigen but by a stimulating substance. When an antigen was only added, differentiation into plasma cells was identified but maturation to B cells was not, and thus it was suggested that differentiation into plasma cells is mainly caused by antigen stimulation. Whether the antigen was only added or the stimulating substance was only added, B cells were found to grow equally, and thus it was suggested that the growth of B cells is caused by the adjuvant. This is also endorsed from that fact that the structure of an adjuvant is the same as the terminal structure of LPS and that cell growth is caused by LPS.

Since the percentage of plasma cells obtained is also increased when a stimulating substance was only added, it was suggested that differentiation occurs in an antigen-independent manner.

B cells in the spleen mature by an antigen or a stimulating substance in a sequence T1-B cells → T2-B cells → B2 cells → activated B cells, and differentiate into plasma cells. In fact, the current method was demonstrated to cause a further maturation of B cells, in particular, compared to when no B2 cells were immunized, they were markedly increased.

Also, since the presence or absence of immunization did not greatly affect the percentage of activated B cells, it was suggested that differentiation from activated B cells into plasma cells occurs very rapidly. When both an antigen and a stimulating substance were added, the percentage of B2 cells was increased compared to when the stimulating substance was only added. This suggested that by adding an antigen simultaneously, the antigen-dependent activation of B cells takes precedence, whereas the antigen-independent activation becomes decreased. Thus, it was suggested that the current in vitro immunization can preferentially differentiates the cells that are producing the antibody of interest.

### Example 9

The present example describes an experimental method of investigating the amounts expressed of markers for B cell differentiation.

BALB/c mice (4 week-old, female, SPF/VAF) were sacrificed by cervical dislocation. After visual identification, the spleens of the mice were removed and washed in 70% ethanol. Furthermore, after washing in PBS, the spleens from two animals were placed on a cell strainer, and mashed with a cell scraper. By washing the cell strainer with 4 mL of PBS(-) for three times, spleen cells were eluted from the cell strainer. They were centrifuged (1300 rpm × 3 min, 4°C) and the cells were collected.

After suspending the cells in 3 mL of the ACK lysing buffer, 10 mL of PBS(-) was added to remove erhythrocytes, centrifuged (1300 rpm × 3 min, 4°C) and the cells were collected. The spleen cells were suspended in 10 mL of the RPMI1640 medium, and by passing them through a cell strainer, insoluble fats, etc., were removed. The cells were counted using a hematocytometer and then concentrated to 1 × 10⁷ cells/mL.

Using a purified hS100A10 protein at 1 nmol as the antigen, 2 × 10⁷ spleen cells were immunized. At this time, 100 µg of muramyl dipeptide (SIGMA) was added as an adjuvant. The immune reaction was conducted by allowing to stand at room temperature for 15 minutes. The stimulating substances (IL-4, IL-5, anti-CD38 antibody, anti-CD40 antibody, LPS) were prepared at 1000 times that of the normal final concentrations. Ten µl of this stimulating substance and the immunized spleen cells (2 mL) were mixed, and adjusted to a concentration of 2 × 10⁶ cells/mL with a RPMI1640 medium containing 40% FBS, and cultured for four days in a 37°C CO₂ incubator. As the control, the one in which the antigen was only added, the one in which the stimulating substance was only added, and the one in which none were added was prepared.

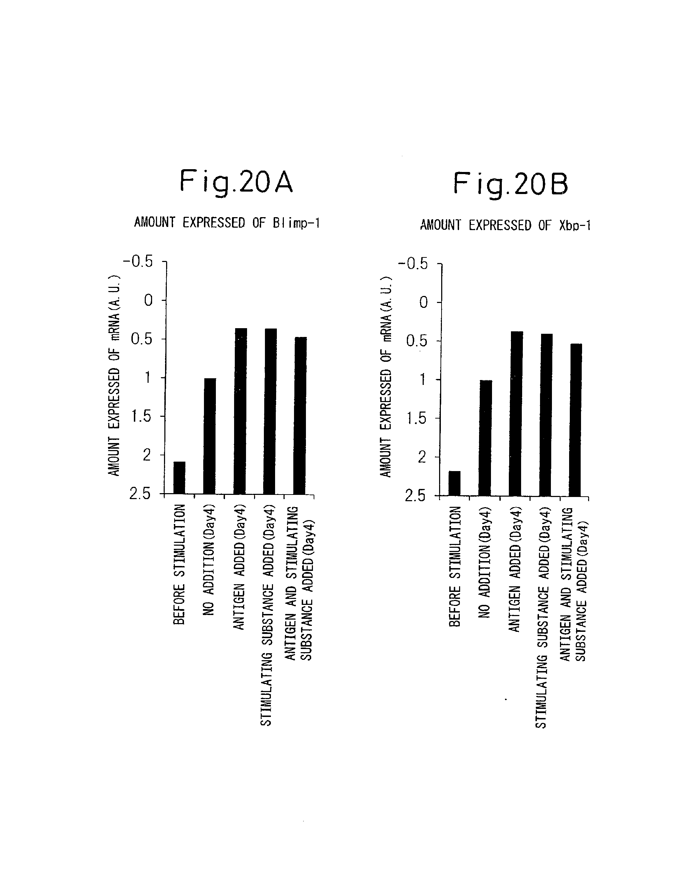

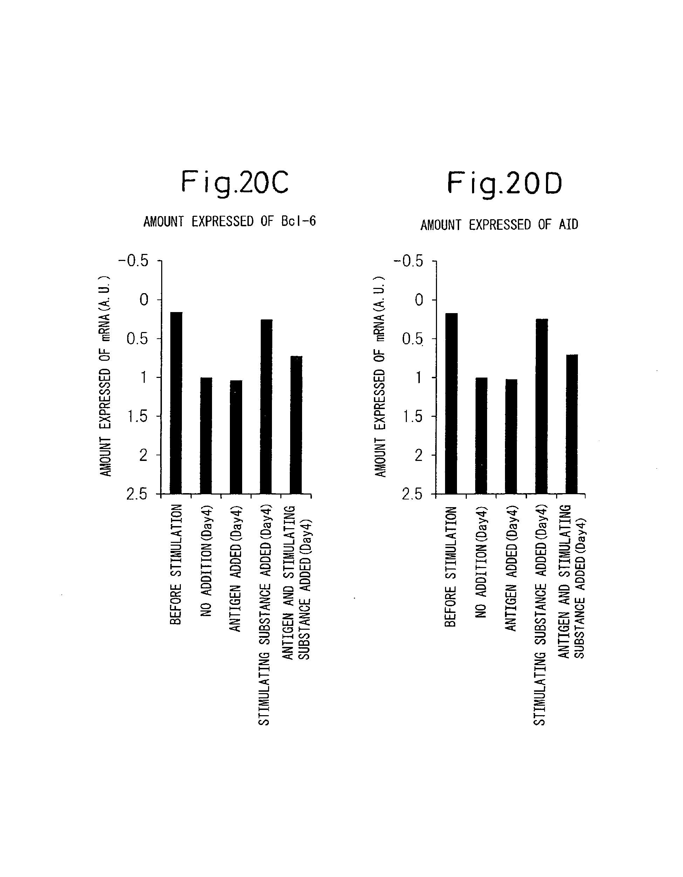

After culturing, all the cells were collected, and using ISOGEN (manufactured by Nippon Gene) total RNA was collected. With 600 ng of this total RNA as the template, a reverse transcription reaction was conducted using the ReverTra Ace (TOYOBO). The reaction composition was a volume of 20 µl according to the manufacturer's protocol, and as the primer, The Oligo(dT)20 (TOYOBO) was used. The reaction condition was 42°C for 20 minutes, 99°C for 5 minutes, and 4°C for 5 minutes. With 1 µl of this product as the template, real-time PCR was conducted using the PowerSYBR Green PCR Master Mix (Applied Biosystems). The reaction condition comprised 95°C for 10 minutes to 40 cycles of 95°C for 15 seconds and 60°C for 1 minute. The equipment used in this example is the 7500Fast (Applied Biosystems). By multiplying the numerical value of the internal standard, P actin, by the percentage of the simultaneously determined plasma cells or activated B cells, the amounts expressed of β actin derived from respective cells were obtained. The amount expressed of plasma cell-derived β actin was used to normalize the amounts expressed of Blimp-1 and Xbp-1, and the amount expressed of activated B cell-derived β actin was used to normalize the amounts expressed of Bcl-6 and AID, respectively. These values were further normalized using the value at no addition and made into graphs. The results obtained are shown in Figure 20A and Figure 20D.

As can be seen from a series of graphs indicating the results of the amounts expressed of markers for B cell differentiation, similarly to the results for antibody staining, the amounts expressed of Blimp-1 and Xbp-1, markers of differentiation into plasma cells, were increased when the antigen was added. Also, Bcl-6 and AID, markers for somatic mutation, were found to be highly expressed when the stimulating substance was added. This confirmed that by adding not only an antigen but also a stimulating substance, an antibody of higher affinity may be easily obtained.

Furthermore, since when an antigen and a stimulating substance were added together, the amounts expressed of markers for somatic mutation are decreased than when the stimulating substance was only added, the presence of a system was suggested in which the coexistence of a specific antigen leads to a slight inhibition of somatic mutation and prevention of activity reduction due to excessive mutation.

Whereas differentiation markers are scarcely expressed in the spleen cells prior to immunization, markers for somatic mutation exhibited high expression. This is probably be due to the constant reaction of increasing antibody repertoire in somatic mutation.

### Example 10

The present example describes an experimental method on ELISA.

After culturing and collecting cells in 5 mL LB medium, they were suspended in 1 mL of PBS and subjected to ultrasonic breaking to collect a supernatant fraction containing soluble MBP-scFv.

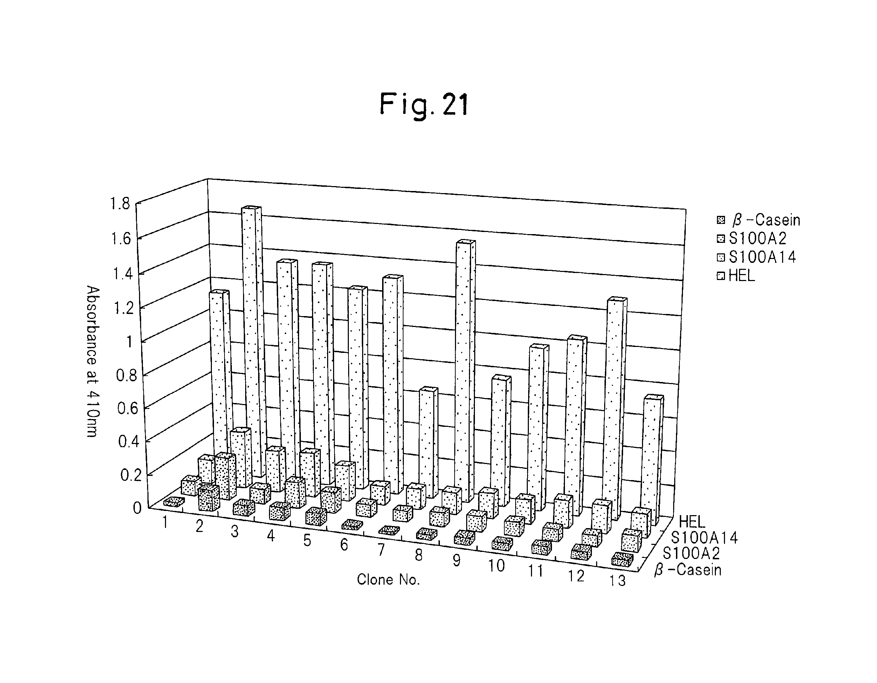

This supernatant fraction was diluted 50-fold in 3% BSA/PBS, and 50 µl of it was used in the ELISA method. The results obtained are shown in Figure 21 to Figure 24.

In the ELISA method shown in Figure 21, according to an in vitro immunization, the activity of anti-HEL/MBP-scFv constructed by immunization with Hen Egg Lysozyme (HEL) as a model antigen was determined by ELISA. In order to investigate antigen specificity, ELISA plates coated with β-casein, S100A2, S100A14 and Hen Egg Lysozyme, respectively, were used in the determination. The result confirmed that by using the present method, scFv clearly specific for Hen Egg Lysozyme can be obtained.

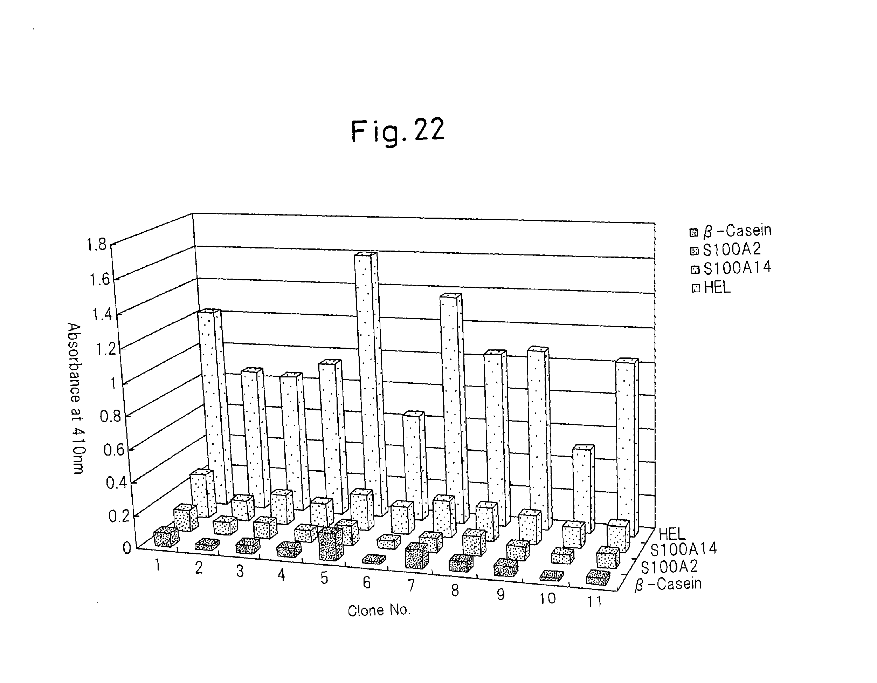

In the ELISA method shown in Figure 22, as a variation of the present in vitro immunization, splenocytes were replaced with MACS for separation. B cells were immunized with the Hen Egg Lysozyme. Similarly to Figure 21, the specificity of the constructed anti-HEL/MBP-scFv was determined, and scFv having a sufficient specificity was identified. This indicates that a directly effective immunization procedure is also possible for the separated B cells.

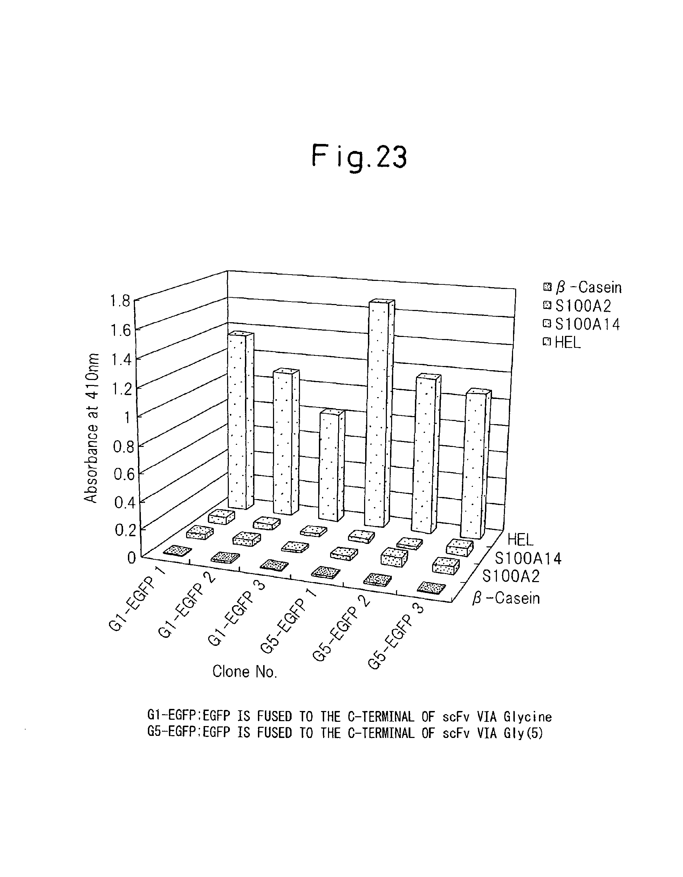

In the ELISA method shown in Figure 23, the gene sequence of scFv of interest of the present in vitro immunization can be obtained. By utilizing this, a function may be further added to scFv. As an example, anti-HEL/MBP-scFv-EGFP was constructed in which EGFP, a fluorescent protein, was fused to the C-terminal of anti-HEL/MBP-scFv. In between scFv and EGFP, two types linkers of different lengths such as Gly and Gly-GLy-Gly-Gly-GLy were used. Similarly to Figure 21, the specificity of MBP-scFv-EGFP constructed was determined, and it was determined possible to prepare a fluorescent scFv having a specificity for the Hen Egg Lysozyme.

As a model antigen other than protein, peptide were immunized according to the present method. In a conventional immunization method, a low molecular weight substance such as a peptide is conjugated to a protein such as KLH and BSA and used as the antigen. In the present method, however, since B cells can be directly targeted in vitro, peptide per se is believed to cause an immunization reaction. Thus, 4 types of peptides of different length were used as the model antigen for direct immunization of peptides. The peptides used are as follows: β-casomorphin-7 (bovine; YPFPGPI, 7 a.a.), angiotensin I (human; DRVYIHPFHL, 10 a.a.), parathyroid hormone; PTH (human; EADKADVNVLYKAKSQ, 16 a.a.). Each peptide was dissolved in PBS, and 10 µg of each was immunized.

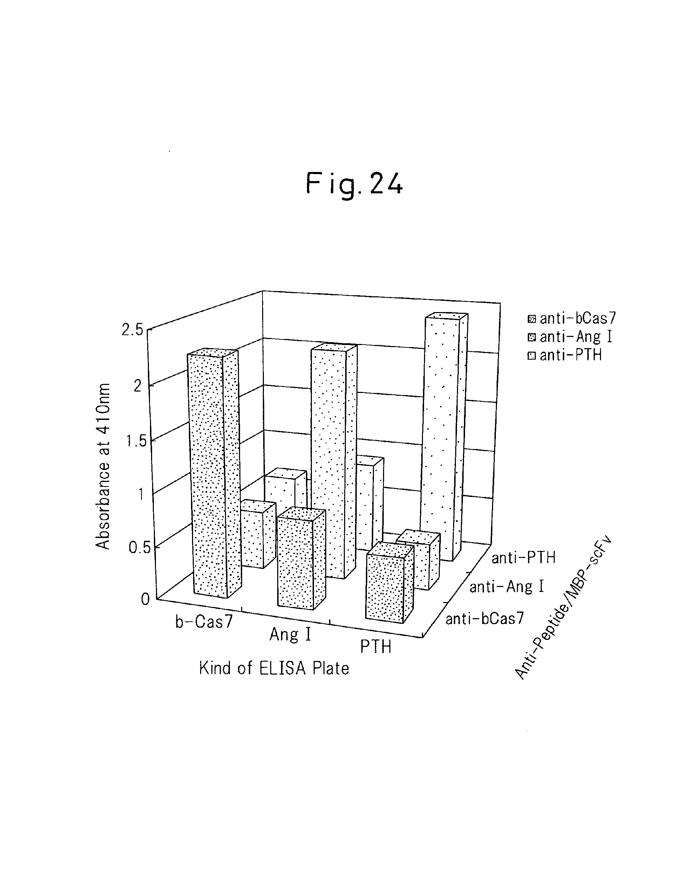

In the ELISA method shown in Figure 24, the activity of anti-β-casomorphin7/MBP-scFv, anti-angiotensin I/MBP-scFv, and anti-PTH/MBP-scFv constructed according to the present method was determined by ELISA. In order to investigate specificity, ELISA plates coated with β-casomorphin-7, angiotensin I, and PTH, respectively, were used in the determination. The result confirmed that scFv significantly specific for the respective peptide can be obtained. It demonstrated that in the present method, scFv can be obtained for a short peptide by merely adding a peptide per se as the antigen.

### Example 11

The present example describes an experimental method of obtaining an anti-mouse protein antibody.

BALB/c mice (4 week-old, female, SPF/VAF) were sacrificed by cervical dislocation. After visual identification, the spleens of the mice were removed and washed in 70% ethanol. Furthermore, after washing in PBS, the spleens from two animals were placed on a cell strainer, and mashed with a cell scraper. By washing the cell strainer with 4 mL of PBS(-) for three times, spleen cells were eluted from the cell strainer. They were centrifuged (1300 rpm × 3 min, 4°C) and the cells were collected.

After suspending the cells in 3 mL of the ACK lysing buffer, 10 mL of PBS(-) was added to remove erhythrocytes, centrifuged (1300 rpm × 3 min, 4°C) and the cells were collected. The spleen cells were suspended in 10 mL of the RPMI1640 medium, and by passing them through a cell strainer, insoluble fats, etc., were removed. The cells were counted using a hematocytometer and then concentrated to 1 × 10⁷ cells/mL.

A purified hS100A10 protein and a purchased mAlbumin (SIGMA) were used as the antigen. Using 1 nmol and 10 nmol of each, 2 × 10⁷ spleen cells were immunized. At this time, 100 µg of muramyl dipeptide (SIGMA) was added as an adjuvant. The immune reaction was conducted by allowing to stand at room temperature for 15 minutes. The stimulating substances (IL-4, IL-5, anti-CD38 antibody, anti-CD40 antibody, LPS) were prepared at 1000 times that of the normal final concentrations. Ten µl of the stimulating substance and the immunized spleen cells (2 mL) were mixed, and the cell concentration was adjusted to 2 × 10⁶ cells/mL with a RPMI1640 medium containing 40% FBS, and cultured for four days in a 37°C CO₂ incubator.

After culturing, all of the cells were collected, and using ISOGEN (manufactured by Nippon Gene) total RNA was collected. With 600 ng of this total RNA as the template, a reverse transcription reaction was conducted using the ReverTra Ace (TOYOBO). The reaction composition was a volume of 20 µl according to the manufacturer's protocol, and as the primer, The Oligo(dT)20 (TOYOBO) was used. The reaction condition was 42°C for 20 minutes, 99°C for 5 minutes, and 4°C for 5 minutes. Using 10 µl of this product as the template, the hypervariable regions of antibody H chain and L chain were amplified by PCR. For PCR, the PrimeSTAR HS (TaKaRa) was used and the reaction composition and reaction condition were as described in the manufacturer's protocol. 400 bp fragments obtained were separated on agarose gel electrophoresis (2.0% Seakem GTG Agarose (TaKaRa)/TAE buffer, 150 V, 30 min), and extracted and purified using the QIAEX II Gel Extraction kit (QIAGEN). The experimental method used was as described in the manufacturer's protocol. With 50 ng of each fragment extracted as the template, a 800 bp scFv fragment was prepared by PCR. For PCR, the PrimeSTAR HS (TaKaRa) was used and the reaction composition and reaction condition were as described in the manufacturer's protocol. A 800 bp fragment obtained was cleaved with restriction enzymes KpnI and HindIII (both manufactured by TOYOBO), and subcloned to a pMa1-c2E vector (NEB).

Using the subcloned DNA, Escherichia coli strain JM109 was transformed, and the clone obtained was cultured in 5 mL of a LB medium supplemented with Overnight Express Auto System (Novagen). After culturing overnight at 27°C, cells were collected from 1.5 mL of the culture liquid, and soluble protein was collected with 300 µl BugBuster Protein Extraction Reagent containing 1 mM AEBSF (SIGMA).

Five µl of the collected soluble protein was subjected to the SDS-PAGE method (10% polyacrylamide gel electrophoresis, 25 mA, 60 min) to identify the expression of MBP-scFv.

Using an ELISA method, the affinity of antibody was examined. As a plate for ELISA, a plate in which mS100A10 or mAlbumin was immobilized was prepared. One µg of protein was added per well, and immobilized overnight at 4°C. After washing in PBS(-), the mS100A10 plate was blocked with 3% BSA (bovine serum albumin (Wako Pure Chemical Industries, Ltd.)) and the mAlbumin plate was blocked with 1% HEL (hen egg white lysozyme (Seikagaku Kogyo)), respectively. After blocking for 1 hour at room temperature, the plate was washed with PBS. To the plate thus prepared, a MBP-scFv antibody sample was added. The antibody to be added to the mS100A10 plate was diluted 50-fold with 3% BSA/PBS(-) and the antibody to be added to the mS100A10 plate with 1% HEL/PBS(-), and allowed to stand at room temperature fro 30 minutes. Fifty µl of this sample was added to a plate, which was allowed to react at 37°C for 1 hour. After washing in PBS(-), 50 µl of a solution in which the secondary antibody (anti-MBP, HRP conjugate (NEB)) was diluted 2000-fold in PBS(-) was added, and allowed to react at 37°C for 1 hour. After washing in PBS(-), 100 µl o the SureBlue Reserve (KPL) was added for color development. The color development reaction was conducted at room temperature for three minutes, and 100 µl of 1N HCl (Wako Pure Chemical Industries, Ltd.) was added to stop the reaction. Subsequently, absorbance at 450 nm/655 nm was determined using a microplate reader model 680 (BioRad)).

From the measured value, a blank value was subtracted, and made into a graph. The ordinate of the graph represents absorbance and the abscissa represents the sample No. A clone for which mAlbumin was immunized was expressed as a Positive value for the value obtained with the mAlbumin plate, and a clone for the value obtained with the mS100A10 plate as a Negative value. A clone for which a difference was specifically observed was indicated by an asterisk.

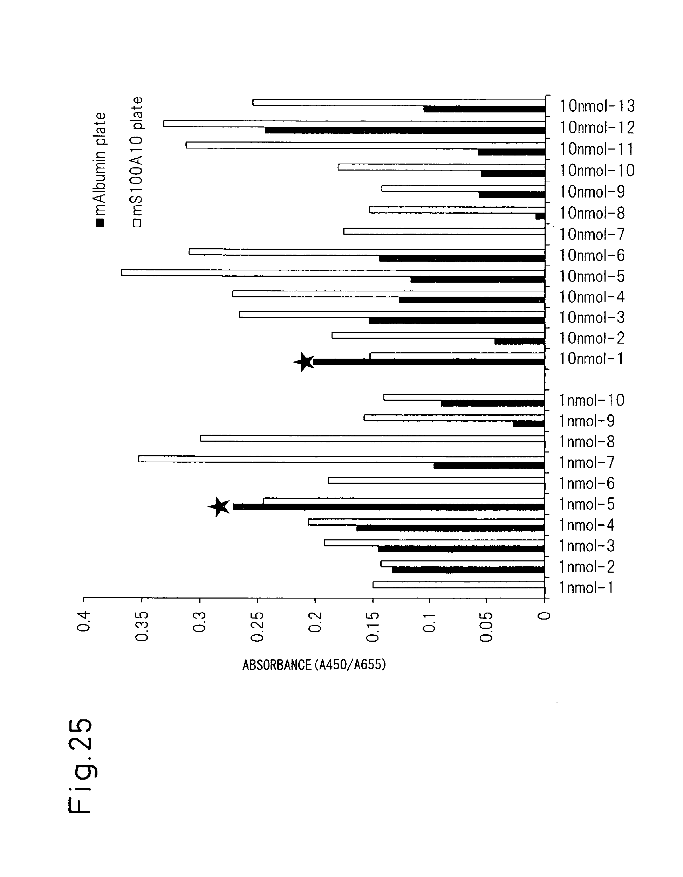

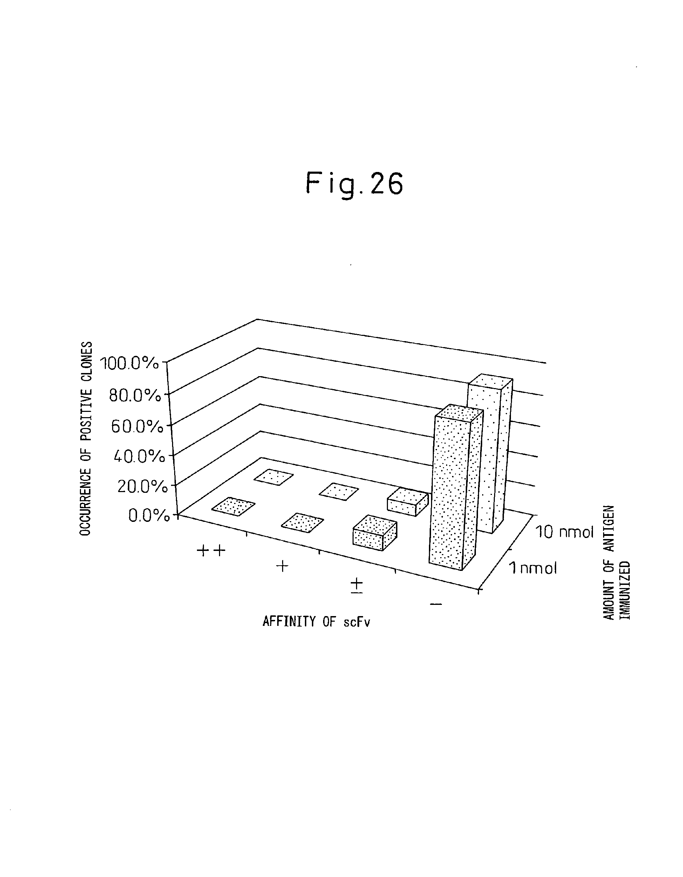

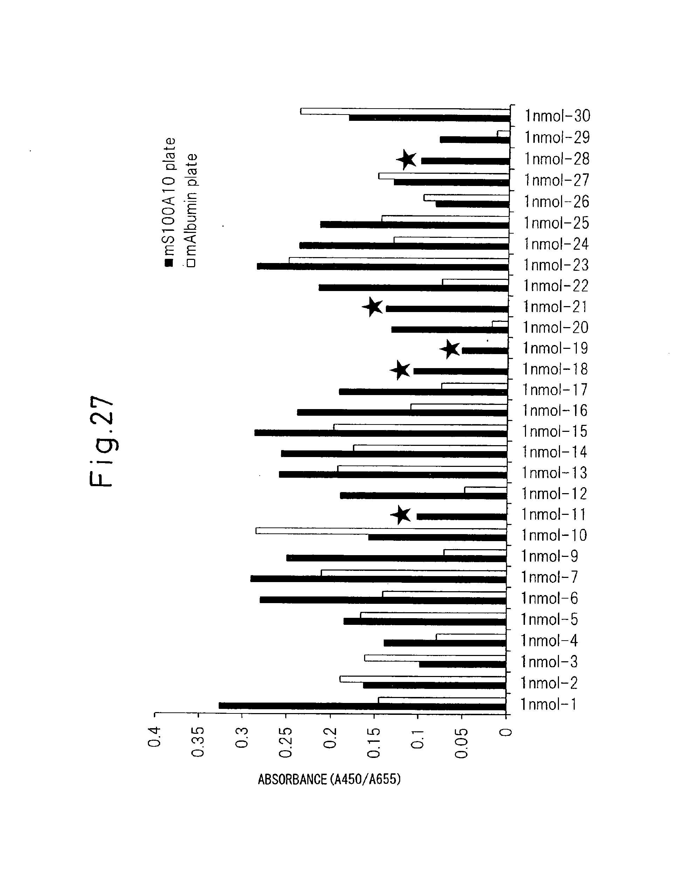

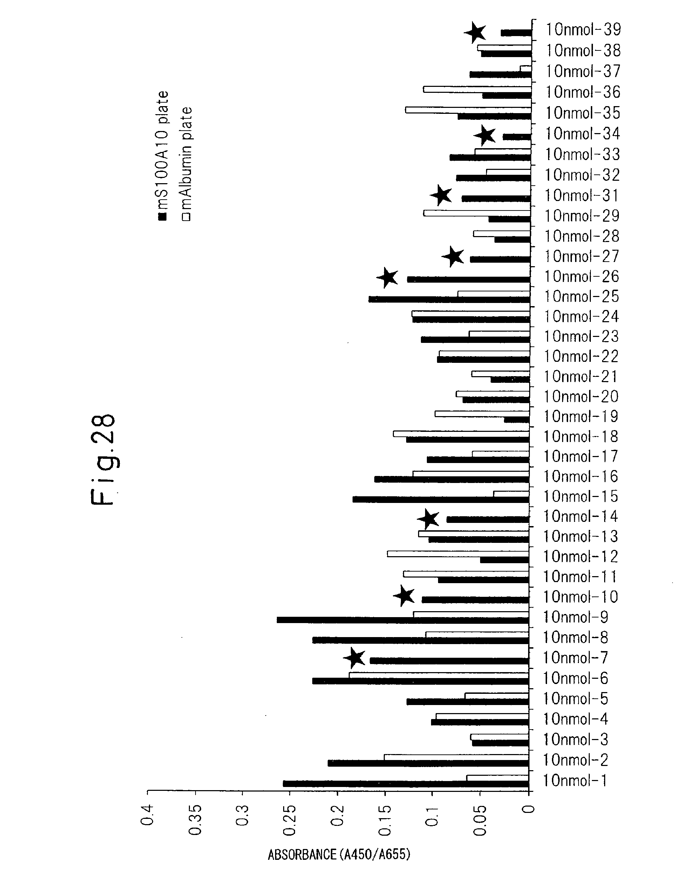

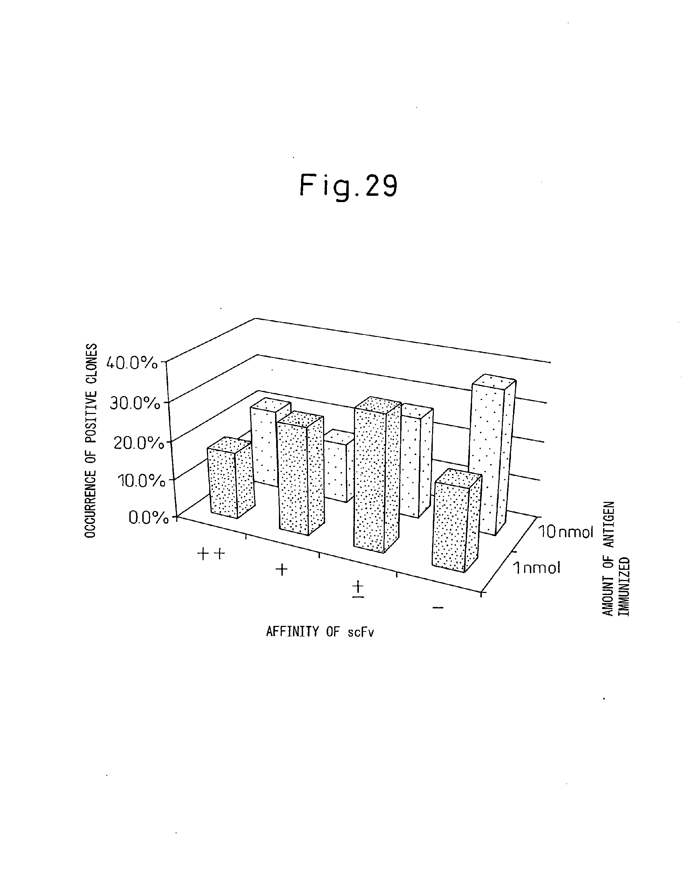

The Positive value was divided by the Negative value to represent a negative value, and a clone for which the Positive value represented a positive value was defined as ++, a clone for which the Positive value represented a value of 2 or greater as +, a clone for which the Positive value represented a value of 1-2 as +/-, and a clone for which the Positive value represented a value of 1 or less as -, and the positivity for each was made into a graph. The results thus obtained are shown in Figure 25 to Figure 29.

As can be seen from a series of graphs that show the result of obtaining anti-moouse protein antibodies, it was possible to obtain a number of antibodies for mS100A10. When the affinity distribution of antibody was examined, a number of clones having no affinity were obtained by immunizing a high concentration of antigen. However, since the positivity of high-affinity antibodies was not greatly different from when immunized with a low concentration of antigen, it is believed that the increased frequency in somatic mutation led to an increase in clones that cannot react to the antigen. This suggested that by using the highest possible concentration when using an antigen having a low antigenicity, antibody that is more suitable for the purpose could be obtained.

On the other hand, it was found very difficult to obtain antibody to mAlbumin. A possible reason for this is that if antibody to this tends to be easily produced, the likelihood of developing an autoimmune disorder may rapidly increase since mAlbumin is abundantly present in the mouse blood. Thus, it is believed that immunocompetent cells have already been selected before being transported from the bone marrow to the spleen, thereby eliminating possible clones. Thus, in producing antibodies to them, it is possible to obtain immunocompetent cells from the bone marrow followed by immunization which may give antibodies more efficiently. Furthermore, the result of obtaining the mS100A10 antibody suggested that the antibody may be more efficiently obtained by immunizing at a concentration higher than 10 nmol. Also, weak as it may be, an antibody to mAlbumin has been obtained, and thus a method of obtaining an antibody of interest by artificially introducing mutation to a gene may be adopted.

### Example 12

The present example describes the obtainment of an anti-low molecular weight-compound antibody and an experimental method.

BALB/c mice (4 week-old, female, SPF/VAF) were sacrificed by cervical dislocation. After visual identification, the spleens of the mice were removed and washed in 70% ethanol. Furthermore, after washing in PBS, the spleens from two animals were placed on a cell strainer, and mashed with a cell scraper. By washing the cell strainer with 4 mL of PBS(-) for three times, spleen cells were eluted from the cell strainer. They were centrifuged (1300 rpm × 3 min, 4°C) and the cells were collected.

After suspending the cells in 3 mL of the ACK lysing buffer, 10 mL of PBS(-) was added to remove erhythrocytes, centrifuged (1300 rpm × 3 min, 4°C) and the cells were collected. The spleen cells were suspended in 10 mL of the RPMI1640 medium, and by passing them through a cell strainer, insoluble fats, etc., were removed. The number of cells was counted using a hematocytometer and then concentrated to 1 × 10⁷ cells/mL.

As the antigen, the HEL-Cy3 protein and HEL-ITC were used. HEL (Seikagaku Kogyo) of them was labelled with the Cy3 Mono Reactive Dye Pack (GE Healthcare) and FITC (Funakoshi Corporation), respectively. The rate of labelling was determined, and using a 1 nmol equivalent of a low molecular weight-compound, 2 × 10⁷ spleen cells were immunized. At this time, 100 µg of muramyl dipeptide (SIGMA) was added as an adjuvant. The immune reaction was conducted by allowing to stand at room temperature for 15 minutes. The stimulating substances (IL-4, IL-5, anti-CD38 antibody, anti-CD40 antibody, LPS) were prepared at 1000 times that of the normal final concentrations. Ten µl of the stimulating substance and the immunized spleen cells (2 mL) were mixed, and the cell concentration was adjusted to 2 × 10⁶ cells/mL with a RPMI1640 medium containing 40% FBS, and cultured for four days in a 37°C CO₂ incubator.

After culturing, all the cells were collected, and using ISOGEN (manufactured by Nippon Gene) total RNA was collected. With 600 ng of this total RNA as the template, a reverse transcription reaction was conducted using the ReverTra Ace (TOYOBO). The reaction composition was a volume of 20 µl according to the manufacturer's protocol, and as the primer, The Oligo(dT)20 (TOYOBO) was used. The reaction condition was 42°C for 20 minutes, 99°C for 5 minutes, and 4°C for 5 minutes. With 10 µl of this product as the template, the hypervariable regions of antibody H chain and L chain were amplified by PCR. For PCR, the PrimeSTAR HS (TaKaRa) was used and the reaction composition and reaction condition were as described in the manufacturer's protocol. 400 bp fragments obtained were separated by agarose gel electrophoresis (2.0% Seakem GTG Agarose (TaKaRa)/TAE buffer, 150 V, 30 min), and extracted and purified using the QIAEX II Gel Extraction kit (QIAGEN). The experimental method was as described in the manufacturer's protocol. With 50 ng of each fragment extracted as the template, a 800 bp scFv fragment was prepared by PCR. For PCR, the PrimeSTAR HS (TaKaRa) was used and the reaction composition and reaction condition were as described in the manufacturer's protocol. A 800 bp fragment obtained was cleaved with restriction enzymes KpnI and HindIII (both manufactured by TOYOBO), and subcloned to a pMa1-c2E vector (NEB).

Using the subcloned DNA, Escherichia coli strain JM109 was transformed, and the clone obtained was cultured in 5 mL of a LB medium supplemented with the Overnight Express Auto System (Novagen). After culturing overnight at 27°C, cells were collected from 1.5 mL of the culture liquid, and soluble protein was collected with 300 µl of the BugBuster Protein Extraction Reagent containing 1 mM AEBSF (SIGMA).

Five µl of the collected soluble protein was subjected to SDS-PAGE method (10% polyacrylamide gel, 25 mA, 60 min) to identify the expression of MBP-scFv.

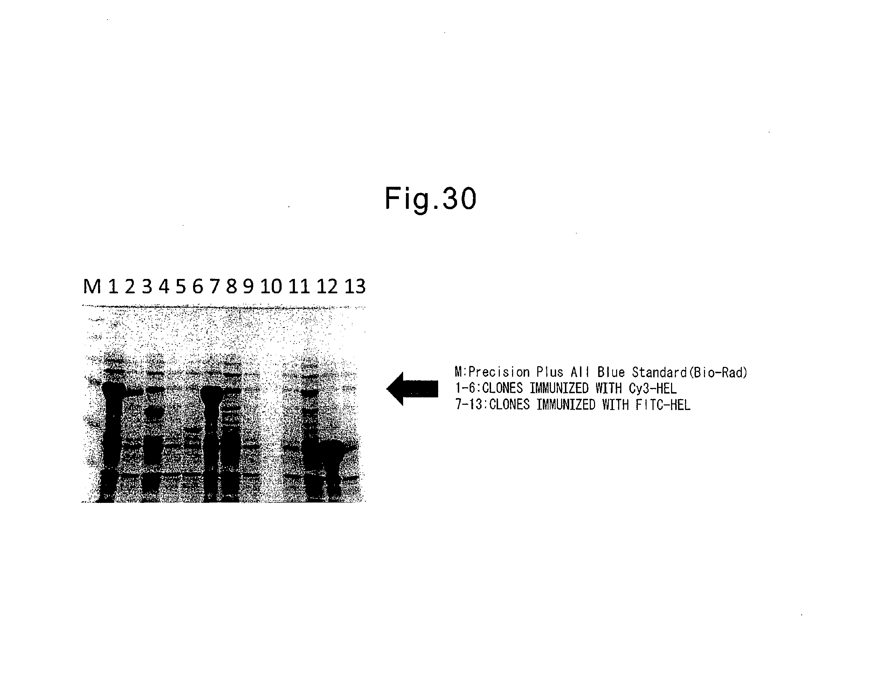

Figure 30 represents an electrophoretogram that shows the obtainment of an anti-low molecular weight-compound antibody. Together with Cy3-HEL and FITC-HEL, the high expression of the protein of interest (arrowed part in the figure) was identified. This suggested that the antibody of interest was obtained.

### Industrial Applicability

In accordance with the present invention, as described above, the generation of an antibody for use in the diagnosis of diseases based on antigen-antibody reaction can be effected in large quantities and in a short period of time, and thus it can promote the expansion of the field of immunological marker testing. Also, antibodies for use in the treatment of diseases based on antigen-antibody reaction can be obtained in various types in a short period of time, the expansion of the field of antibody pharmaceuticals can be promoted.

Furthermore, the generation of an antibody that enables the detection of the S100A10 protein, a renal cancer marker, can be effected in large quantities and in a short period of time, the cost of antibody production could be lowered, and thus urine testing for the purpose of cancer screening could be carried out more frequently and at low cost.

## Claims

1. A method of generating an antibody comprising the steps of:
immunizing a tissue containing cells for immunization in vitro in a culture liquid containing an antigen and a stimulating substance,
selecting the immunized cells, and
obtaining an antibody from the above selected immunized cells.

2. The method of generating an antibody according to claim 1, said method further comprising the steps of obtaining an amplified antibody gene by a PCR amplification means and then expressing and preparing the antibody or a protein similar to the antibody by host cells.

3. The method of generating an antibody according to claim 1 wherein said tissue is an animal for immunization.

4. The method of generating an antibody according to claim 1 wherein said selected cells are cells that underwent class switching or affinity maturation.

5. The method of generating an antibody according to claim 1 wherein the selection of said immunized cells is based on flow cytometry or staining.

6. The method of generating an antibody according to claim 1 wherein said stimulating substance is a substance that stimulates a cytokine receptor, a surface antigen or a receptor involved in signal transduction expressed on immunocompetent cells.

7. The method of generating an antibody according to claim 1 wherein said antigen is a peptide.

8. The method of generating an antibody according to claim 1 wherein said antigen is a protein belonging to the S100 family, and a single chain antibody scFv (single chain variable fragment) or an antibody IgG1 (immunoglobulin G1) are obtained.

9. The method of generating an antibody according to claim 1 wherein said antigen is a protein belonging to the S100A10 family, and a single chain antibody scFv (single chain variable fragment) or an antibody IgG1 (immunoglobulin G1) are obtained.

10. The method of generating an antibody according to claim 6 wherein said stimulating substance is at least one of IL-4 or Il-5 as a stimulating substance for a cytokine receptor, anti-CD38 antibody or anti-CD40 antibody as a stimulating substance for a surface antigen, and a LPS (lipopolysaccharide) as a stimulating substance for a receptor involved in signal transduction.

11. The method of generating an antibody according to claim 1 wherein an antibody is generated by immunizing in vitro a tissue containing cells for immunization in a culture liquid containing one or more than one stimulating substance selected from IL-4, IL-5, anti-CD38 antibody, anti-CD40 antibody and LPS (lipopolysaccharide).

12. The method of generating an antibody according to claim 1 further comprising the steps of:
determining the amino acid sequence of said selected antibody, and
comparing this amino acid sequence with an amino acid sequence library of human antibody to select or modify a sequence with a high homology from the amino acid sequence library of human antibody to generate a humanized antibody.
